# EUROPÄISCHE PATENTSCHRIFT

(11) **EP 3 918 237 B1**
(45) Veröffentlichungstag und Bekanntmachung des Hinweises auf die Patenterteilung: **02.09.2026**
(21) Anmeldenummer: 20705293.7
(22) Anmeldetag: 28.01.2020
(51) Int. Cl.: A61M 60/196, A61M 60/258, A61M 60/268, A61M 60/427, A61M 60/585, A61M 60/837, A61M 60/894, A61M 60/896

(54) **KÜNSTLICHES HERZSYSTEM**
ARTIFICIAL HEART SYSTEM
SYSTEME CARDIAQUE ARTIFICIEL

(30) Priorität: 28.01.2019 DE 102019000611
(43) Veröffentlichungstag der Anmeldung: 08.12.2021
(73) Patentinhaber: Saadat, Mohammad Mohsen, 59494 Soest (DE)
(72) Erfinder: Saadat, Mohammad Mohsen, 59494 Soest (DE)
(74) Vertreter: BSB Patentanwälte Schütte & Engelen Partnerschaft mbB
(86) Internationale Anmeldenummer: PCT/EP2020/052058
(87) Internationale Veröffentlichungsnummer: WO 2020/157073

(56) Entgegenhaltungen:
- US-A- 4 222 127
- US-A- 4 397 049
- US-A- 4 863 461
- US-A- 5 458 468

## Beschreibung

Die Erfindung betrifft ein künstliches Herzsystem für Menschen und andere Lebewesen umfassend mindestens eine Herzhälfte, die anstelle des biologischen Herzens oder parallel dazu im Körper implementiert wird und als Pumpe mindestens einen Blutkreislauf des Menschen oder eines anderen Lebewesens ganz oder teilweise unterstützend aufrecht hält, und wenigstens eine Antriebseinheit und wenigstens eine Steuereinheit, die vorzugsweise außerhalb des Körpers angeordnet sind.

Es gibt zahlreiche künstliche Herzen, die im Körper eingebaut und mit elektrischer Energie oder von außen mit Druckluft angetrieben werden. Da der Energiebedarf des Herzens so groß ist, dass keine passende Energiequelle dauerhaft im Körper implementiert werden kann, muss die erforderliche Energiequelle außerhalb des Körpers austauschbar und wieder aufladbar platziert werden.

Die vorhandenen Pumpen, Apparate und Aggregate, die das natürliche Herz ersetzen oder unterstützen, sind in der Regel sehr groß, unhandlich und vor allem sehr laut. Darüber hinaus arbeiten diese künstlichen Herzen in der Regel mit technischen Pumpen, die auf die Dauer die Blutkörperchen mechanisch beschädigen oder sie verwenden Membranen aus elastischen Materialien, die mit der Zeit porös und undicht werden und schließlich aufreißen.

DE 689 06 403 T2 zeigt ein künstliches Herz bestehend aus zwei Herzhälften. Jede Herzhälfte besteht aus drei Kammern, zwischen denen zwei elastisch dehnbare Membranen (10, 11) angeordnet sind. Die Antriebspumpe (22) presst bei jeder Herzkontraktion eine Flüssigkeit gegen die Membrane (11), die ihrerseits den Druck auf die Flüssigkeit zwischen den beiden Membranen (10, 11) und von dort auf die Membrane (10) der Blutkammer (9a) weiterleitet. Bei diesem Vorgang fließt einerseits die Flüssigkeit zwischen den beiden elastischen Membranen (10, 11) zurück in den Vorratsbehälter (13) als auch von der Blutkammer (9a) in die Arterie (7) hinein. Beim Ansaugen der Antriebsflüssigkeit durch die Pumpe (22) fließt das Blut von der Venenöffnung (3) in die Blutkammer (9a) und gleichzeitig fließt die Flüssigkeit aus der Vorratskammer (13) zurück in den Raum zwischen den beiden Membranen (10, 11). Damit ist die Aufteilung der beiden Volumina den jeweiligen Fließwiderständen der beiden Flüssigkeiten überlassen und dies für beide Herzhälften jede für sich. Ein definiertes bzw. erforderliches Herzschlagvolumen ist somit nicht gewährleistet. Die Erfindung weist einige gravierende Nachteile und Probleme auf, die bis heute nicht gelöst werden konnten. Beispielsweise liegen zwei Motoren und zwei Pumpen mit ihren Hitze- und Brandgefahren im inneren des Körpers. Die Blutkammer (9a) kann nicht komplett entleert werden, was eine Gefahr für Thrombosenbildung darstellt. Der synchrone Lauf der beiden Motoren ist nicht zwangsläufig gewährleistet und über die Funktionsweise der Herzklappen ist ebenfalls kein Wort geschrieben. Das Blut kommt innerhalb des künstlichen Herzens mit mehreren unterschiedlichen Werkstoffen in Berührung. Die Elektromotoren werden ständig in ihrer Drehrichtung hin- und her umgepolt, was deren Lebensdauer erheblich verkürzt.

DE 698 20 603 T2 zeigt ein künstliches Herz deren Antriebe und Pumpen ebenfalls direkt im Herzen und somit mit allen zuvor genannten Nachteilen unzugänglich im Körper sitzen.

US 4 222 127 A offenbart ein künstliches Herz nach dem Oberbegriff des Anspruchs 1.

Der Erfindung liegt die Aufgabe zugrunde, ein künstliches Herz bereitzustellen, welches die zuvor beschriebenen Nachteile wenigstens teilweise verbessern. Insbesondere soll ein einfaches, leise arbeitendes, leistungsstarkes und vorzugsweise sicheres künstliches Herz zur Verfügung gestellt werden, das vorzugsweise zwecks dauerhafter Aufrechterhaltung oder der Unterstützung mindestens eines der beiden Blutkreisläufe im Körper implementiert oder außerhalb des Körpers von Menschen und anderen Lebewesen angebracht wird, und von einer außerhalb des Körpers platzierten Antriebs- und Steuereinheit so betrieben wird, dass im Notfall an jedem Ort der Patient selbst sich erste Hilfe leisten kann.

Diese Aufgabe wird erfindungsgemäß mit den in Anspruch 1 angegebenen Merkmalen gelöst.

Die Unteransprüche stellen vorteilhafte Weiterbildungen der Erfindung dar.

Das erfindungsgemäße künstliche Herz zur Aufrechterhaltung und/oder Unterstützung wenigstens eines Blutkreislaufes von Menschen und/oder anderen Lebewesen umfasst wenigstens ein und insbesondere zwei hermetisch abgedichtete Herzhälften mit je einer Blutkammer und einer Antriebskammer, wobei jede Herzhälfte eine teilbare harte Schale umfasst. Die Blutkammer wird durch einen Beutel zur Verfügung gestellt, wobei sich der Blutbeutel wenigstens abschnittsweise und insbesondere in etwa bis zur Hälfte bewegungslos an die Wandung der harten Schale anlegt. Die zweite Hälfte des Blutbeutels wird durch den Druck aus der Antriebskammer im Wesentlichen spannungslos und vorzugsweise ohne Knickung mit einer umlaufend abrollenden Biegung wenigstens abschnittsweise und insbesondere komplett in die erste Hälfte hinein verlagert. Weiterhin legt sich der Antriebsbeutel wenigstens abschnittsweise und insbesondere in etwa bis zur Hälfte bewegungslos an die Wandung der harten Schale und liegt mit seiner zweiten Hälfte Rücken an Rücken an den Blutbeutel, und erzeugt vorzugsweise spannungslos und ohne Dehnung oder Knickung den Abrollvorgang der Biegungswelle.

Bevorzugt sind auf der Rückseite der beweglichen Hälfte der Blut- und Antriebsbeutel breitenkreisförmige Zähne wie Wölbungen durch Materialanhäufung und/oder durch Einlegen und/oder Einarbeiten dehnungssteifer Ringe vorhanden, die sich insbesondere kettengliedförmig ineinander begeben.

Besonders bevorzugt ist zwischen den beiden Beuteln ein wenigstens ein elastisches Kissen vorgesehen, insbesondere wenigstens ein linsenförmig konvex geformtes elastisches Kissen.

Vorzugsweise sind die Beutel wenigstens abschnittsweise aus einem biologischen Faden gewebt.

In zweckmäßigen Ausgestaltungen umfasst die bzw. jede harte Schale zwei Halbschalen, die durch eine positiv und negativ formschlüssige lösbare Verbindung mit einer kegelförmigen Schnittstelle ineinander gesteckt werden.

In vorteilhaften Ausgestaltungen befinden sich auf der Innenseite der Herzhälfte bzw. der Schale Längs- und Quernuten, die insbesondere als dritte Kammer für die Aufnahme eines viskösen Schmier- und Abdichtmittels dienen.

Bevorzugt befinden sich auf der Innenseite der Herzhälfte bzw. der Schale Längs- und Quernuten mit einem Anschluss nach außen, die insbesondere als dritte Kammer zur Regulierung des Herzschlagvolumens dienen.

Besonders bevorzugt befinden sich an der Herzhälfte positive und negative Elemente derart, dass eine zweite Herzhälfte in Bezug auf ihre Längsachse um 180° gedreht, an der ersten Herzhälfte befestigt wird und gemeinsam beide Blutkreisläufe des Menschen oder eines anderen Lebewesens als Blutpumpe aufrechterhalten.

Vorzugsweise ist wenigstens ein Antriebsmedium wenigstens eine Flüssigkeit.

In zweckmäßigen Weiterbildungen wird das Antriebsmedium durch eine Zylinder-Kolben-Einheit in den Antriebsbeutel hinein und heraus gepumpt, deren Kolbenstange durch einen Spindeltrieb angetrieben wird, dessen Spindel eine doppelgängige rechts-links Endlosschleife besitzt.

Bevorzugt wird das Antriebsmedium durch eine Zylinder-Doppelkolben-Einheit abwechselnd in die Antriebsbeutel hinein und heraus gepumpt, deren Kolbenstange insbesondere durch einen oder zwei Spindeltriebe angetrieben wird, deren Spindel eine doppelgängige rechts-links Endlosschleife besitzt.

In bevorzugten Ausgestaltungen wird das Antriebsmedium durch zwei Zylinder-Kolben-Einheiten gleichzeitig in die rechte und linke Antriebsbeutel hinein und heraus gepumpt, deren Kolbenstangen durch einen oder zwei Spindeltriebe angetrieben werden, deren Spindel eine doppelgängige, in einer endlosen Schleife rechts und links verlaufende Spiralnut besitzt.

Besonders bevorzugt ist wenigstens ein Ein- und Auslassventil der Blutkammer aus bzw. mit dreiecksförmigen bzw. dreieckigen harten Lamellen vorgesehen, die in runder Anordnung um einen mehreckigen, vorzugsweise sechseckigen Durchgangsöffnung bzw. Bohrung eines Ventilringes durch Drehgelenke schwenkbar angeordnet sind.

Vorzugsweise sind an zwei Seiten der Ventillamellen durch Drehgelenke Seitenlamellen derart gestaltet und angeordnet, dass sie sich beim Schließen des Ventils hinter den Ventillamellen radial nach außen zusammen falten, den Spalt zwischen den Ventillamellen komplett abdichten und den Ventillamellen Biegesteifigkeit verleihen.

Bevorzugt befinden sich anstelle der Seitenlamellen zwischen den Ventillamellen federnde Elemente wie elastische Bügel.

In zweckmäßigen Weiterbildungen befindet sich an dem Ventilring wenigstens ein offenes bzw. strömungsoffenes vorzugsweise pyramidenförmiges Gittergebilde, das als Abstützung und besseren Abdichtung der Ventillamellen dient, deren Öffnungsbegrenzung durch die Anlehnung der buckelartigen Rippen an das Rohrstück erfolgt.

Bevorzugt wird mindestens ein Ventil durch eine Minikamera oder mit einem optischen Sensor überwacht.

Besonders bevorzugt ist mindestens ein Drucksensor vorgesehen, der insbesondere innerhalb der harten Schale angeordnet ist, der den momentanen Blutdruck misst und an eine Steuereinheit weiterleitet.

Zweckmäßig entkoppelt eine mechanische Kupplung bei Bedarf die Verbindung zwischen den Antriebssystemen und den Kolbenstangen vorzugsweise manuell, um die Zylinder-(Doppel)Kolben-Einheiten per Hand zu betätigen und vorzugsweise nach Aufhebung der Störung wieder per Knopfdruck oder anders die Verbindung herzustellen bzw. die Kupplung einzukoppeln.

Vorzugsweise sind die Außenseite der Anschlüsse der Herzhälfte mit positiven Wölbungen versehen und die Arterie, Vene oder der Anschlussschlauch werden mit Hilfe wenigstens eines Schnellverschlusses vorzugsweise mit Widerhacken und/oder negativ gewölbter Oberfläche und insbesondere mit wenigstens einer Sicherung vorzugsweise locker und rutschfest befestigt.

Das erfindungsgemäße Herz umfasst wenigstens eine und vorzugsweise zwei Herzhälften. Jede Herzhälfte ist hohl und besitzt eine aus mindestens zwei Teilen bestehende harte Schale, die hermetisch luft- und flüssigkeitsdicht verschlossen ist. In jeder Herzhälfte befinden sich mindestens zwei Kammern, eine Blutkammer und eine Antriebskammer. Die Blutkammer besteht vorzugsweise komplett aus einem Blutbeutel, dessen rhythmische Pulsation durch die Antriebskammer den Blutstrom eines der beiden Blutkreisläufe, den Blutkreislauf durch die Lunge oder den Blutkreislauf durch den restlichen Körper, aufrechterhält. Der Blutbeutel füllt im befüllten Zustand im Wesentlichen das gesamte Volumen und insbesondere das gesamte Volumen einer Herzhälfte. Die Antriebskammer besteht ebenfalls vorzugsweise komplett aus einem Antriebsbeutel, dessen Volumen im befüllten Zustand im Wesentlichen und insbesondere vollständig dem Volumen einer Herzhälfte entspricht und sich gegenüber dem Blutbeutel befindet.

Die harte Schale jeder Herzhälfte ist vorzugsweise dreidimensional elliptisch gestaltet und besitzt vorzugsweise an wenigstens einer ihrer Außenlängsseiten Befestigungselemente in Form von Steckverbindungen, die es ermöglichen, zwei identische oder gleiche Herzhälften in Bezug auf eine vertikale Achse parallel zum Brustbein um 180° spiegelbildlich gedreht, anzuordnen und formschlüssig ineinander zu stecken. Die Steckverbindungen der beiden Herzhälften besitzen parallel zu ihrer vertikalen Spiegelachse Filmgelenke, die eine elastische Winkeländerung der beiden Herzhälften zueinander ermöglichen.

Bevorzugt wird durch das Ineinanderstecken von positiv und negativ profilierten Befestigungselementen oder miteinander Verschrauben von zwei identischen oder gleichen Herzhälften ein ganzes Herz zusammengesetzt, wobei nach wie vor jede Herzhälfte für sich eine eigene geschlossene harte Schale und darin befindlich eine Blutkammer und eine Antriebskammer besitzt.

Ein derart konstruiertes künstliches Herz besitzt vorzugsweise die geometrische Form und Größe eines natürlichen Herzens, vorzugsweise jedoch flacher. Eine Herzhälfte besitzt vorzugsweise die geometrische Form eines Ellipsoids und sieht aus wie ein Ei, das an seinem Äquator oval zusammengedrückt ist.

Die Wandstärke der harten Schale jeder Herzhälfte beträgt vorzugsweise ein paar Zehntel Millimeter. Sie besteht aus mindestens zwei Teilen, vorzugsweise aus zwei Halbschalen, deren Trennlinie beliebig, längs durch die Pole, schräg diagonal oder wie im Folgenden bildlich dargestellt quer am Äquator verläuft. Die Trennlinie zwischen den beiden Halbschalen am Äquator oder etwas schräg dazu, bildet eine Ellipse bis zu einem Kreis, deren oder dessen eingespannte Ebene in etwa senkrecht zur Hauptachse der harten Schale bzw. etwas geneigt dazu steht. Die beiden Halbschalen werden mit den sich in ihnen befindlichen Blut- und Antriebskammern luft- und flüssigkeitsdicht ineinander gesteckt oder miteinander verschraubt oder durch Klammern zusammengehalten. Die beiden Halbschalen sind bevorzugt an ihren Trennkanten innenseitig mit einem Absatz so versehen, dass die beiden Absätze nach dem Zusammenfügen der beiden Halbschalen in ihrem Innenraum entlang ihrer gemeinsamen Trennfüge eine geschlossene und ellipsenförmige Umfangsnut bilden.

Diese Umfangsnut und andere Längs- und Quernuten auf der inneren Oberfläche der beiden Halbschalen, die ähnlich wie Breiten- und Längengrade eines Globus verlaufen, bilden gemeinsam eine dritte Kammer, die Schmierkammer, durch die ein Schmiermittel wie Paraffin rundherum zwischen den beiden Kammern, Blutkammer und Antriebskammer, und der Innenwand der harten Schale hin gelangt.

Die Halbschalen werden besonders bevorzugt aus einem biologisch verträglichen Kunststoff entweder gespritzt und/oder sie werden mit einem dreidimensionalen Druckverfahren aus Kunststoffen und/oder einem Kunststoff-Metall-Gemisch gedruckt bzw. geplottet. Sie erhalten vorzugsweise bei Bedarf eine Oberflächenbeschichtung durch Edelmetallen wie Gold, Platin oder Titan oder eine keramische Beschichtung. Werden sie aus Aluminiumlegierungen hergestellt, können sie elektrochemisch eloxiert (anodizing) werden.

Jede Herzhälfte besitzt im Inneren mindestens zwei, jedoch vorzugsweise drei voneinander unabhängige Kammern, eine Blutkammer für das Blut, eine Druck- und Saugkammer, die als Antriebskammer dient und optional eine dritte Kammer als Zwischenraum zwischen den zuvor genannten Kammern für ein Schmiermittel, dessen Volumen gleichzeitig das Herzschlagvolumen bestimmt. Alle Kammern liegen vorzugsweise in der harten Schale luft- und flüssigkeitsdicht nebeneinander oder übereinander oder relativ zu einander diagonal angeordnet. Die Kammern jeder Herzhälfte können auch koaxialineinander angeordnet werden. Mindestens die Blutkammer ist vorzugsweise aus einem sehr dünnen, biologisch verträglichen, zugfesten, sehr biegsamen, schmiegsamen und widerstandslos verformbaren Material wie der Faden der Seidenraupe oder der Spinne oder aus einem sehr dünnen synthetischen Faden wie Nylon-, PET-, Perlon in Form eines Ballons, eines Kissens wie ein Beutel mit zwei zylinderförmigen Ärmeln aus einem Stück gewebt oder aus PUC in 3D-Verfahren gedruckt.

Die Blut- und Antriebsbeutel bzw. -kissen sind bevorzugt außenseitig mit einer viskosen Flüssigkeit als Schmiermittel wie Paraffin, vorzugsweise aus der dritten Kammer, benetzt. Sie liegen vorzugsweise je zu einer Hälfte an der Wandung ihrer Halbschale, wo sich ihr Anschluss befindet. Die anderen zweiten Hälften liegen bevorzugt Rücken an Rücken mit dem Schmiermittel dazwischen und wandern gemeinsam rhythmisch pulsierend vom Anschluss des Antriebsmediums zu den Blutanschlüssen bzw. zum Aortenventil und zurück. Dabei verändern sie besonders bevorzugt ihr Volumen abwechselnd von nahezu Null bis auf 98% des Volumens der harten Schale, abhängig vom veränderbaren Volumen des Schmiermittels, ohne sich bei dieser Arbeit und Verformung zu dehnen, zu knicken oder zu falten. Die Verformung des Blutbeutels entsteht insbesondere durch eine kreisförmige über die zweite Hälfte wandernde Biegewelle, die bei ca. zwei Drittel der unteren Halbschale beginnt und sich bis zum Äquator der Herzhälfte auf und ab bewegt.

Bevorzugt verringert die viskose Flüssigkeit in der dritten Kammer als Gleitschicht die Reibung zwischen den beiden Kammern und der inneren Wandung der harten Schale, hält das Gewebe der Beutel geschmeidig und dicht und sorgt zusätzlich für eine gleichmäßige Belastung und Druckverteilung der beiden Beutel. Die Menge bzw. das Volumen der viskosen Flüssigkeit zwischen den beiden Kammern dient als Mittel zur Regulierung des Herzschlagvolumens. Zwecks Regulierung der Menge dieser Gleitflüssigkeit und deren Austausch besitzt die dritte Kammer einen Anschluss an der harten Schale, vorzugsweise in der Nähe ihres Äquators. Von diesem Anschluss führt ein dünner Schlauch parallel zum Antriebsschlauch vom Körperinneren nach außen zum Pumpenaggregat und Steuereinheit an einem Korsett, auf dem Brustkorb, am Gürtel oder wo anders am Körper befestigt.

Die Blutkammer besteht vorzugsweise aus einem ellipsoidförmigen, bevorzugt mit einem zylinderförmigen Eingang und einem zylinderförmigen Ausgang wie zwei Ärmeln, vorzugsweise aus einem Stück gewebten Beutel. Der Blutbeutel liegt besonders bevorzugt unmittelbar im Blutkreislauf entweder in der rechten Herzhälfte im sauerstoffarmen Blutkreislauf und pumpt das Blut von den Muskeln und Organen des Körpers kommend zur Lunge oder sie liegt in der linken Herzhälfte im sauerstoffreichen Blutkreislauf und pumpt das Blut von der Lunge kommend zu den Muskeln und Organen des Körpers. Der Blutbeutel liegt insbesondere in der oberen Halbschale und besitzt jeweils eine Bluteintritts- und eine Blutaustrittsöffnung, in denen sich je ein Rückschlagventil und ein optisches System mit einer integrierten Kamera zur Ferndiagnose des Ventils sowie mindestens einen Drucksensor innerhalb der unbeweglichen Hälfte zur Messung des Blutdrucks befinden.

Die beiden Anschlüsse der Blutkammer liegen beim künstlichen Herzen wie bei dem natürlichen im oberen Bereich der harten Schale zum Kopf hin gerichtet. Die Mittelebene zwischen der rechten und der linken Herzhälfte, das Kammerseptum (Septum interventriculare Cordis) steht ca. senkrecht zur Brustebene und ca. 5 bis 15° entgegen dem Uhrzeigersinn geneigt zu einer geraden Wirbelsäule. Bluteintritts- und Blutaustrittsöffnungen liegen bei den beiden Herzhälften in etwa symmetrisch in Bezug auf die beschriebene Mittelebene, wobei die beiden Blutaustrittsöffnungen, die Lungenschlagader und die Aorta, sich am nächsten zur Herzmittelebene und die beiden Bluteintrittsöffnungen, die Hohlvenen und die Lungenvenen, sich etwas entfernter davon befinden. Dadurch lässt sich das komplette künstliche Herz aus zwei gleiche Herzhälften, jeweils um 180° bezüglich der mittleren Längsachse gedreht, zusammensetzen. Somit kann bei Bedarf auch nur eine Herzhälfte ausgetauscht oder parallel zum natürlichen Herzen unterstützend eingesetzt werden. Die Anschlüsse der Blutbeutel werden unmittelbar mit den Adern verbunden, so dass das Blut mit keinem anderen Werkstoff in Berührung kommt außer mit dem Blutbeutel.

Vorzugsweise unterhalb bis neben jeder Blutkammer befindet sich die Antriebskammer. Sie dient zum Entleeren der Blutkammer durch Erzeugung von Druck und wieder Befüllen der Blutkammer durch Erzeugung von Sog innerhalb der harten Schale der Herzhälfte. Die Antriebskammer besteht vorzugsweise ebenfalls aus einem Beutel, der aus einem zugfesten, sehr biegsamen, schmiegsamen, widerstandslos verformbaren Material mit einem zylinderförmigen Anschluss, vorzugsweise aus einem Stück, gewebt ist. Der Anschluss liegt vorzugsweise an der Herzspitze und dient zum Ein- und Ausströmen einer Antriebsflüssigkeit, die vorzugsweise Bestandteil des Blutes, wie z.B. eine Kochsalzlösung oder eine andere mit Blut verträgliche Flüssigkeit wie z.B. Kokosmilch ist. Wie bei dem Blutbeutel liegt auch beim Antriebsbeutel eine Hälfte, hier die untere, stets an der Wandung der unteren Halbschale, während sich ihre obere Hälfte Rücken an Rücken an die untere Hälfte des Blutbeutels anlegt und sich mit ihr auf und ab bewegt.

Eine besonders sichere und langlebigere Ausführung der anmeldungsgemäßen Herzhälfte sieht vor, dass die zugfesten Fäden der beiden Beutel, Blutbeutel und Antriebsbeutel, beim Weben in Umfangs- und in Längsrichtung ähnlich wie Breiten und Längengrade verlaufend gewebt werden. Von jedem Beutel liegt die Hälfte mit dem Anschluss nach außen in seiner Halbschale faltenfrei an der Wandung und die anderen beiden Hälften, die frei sind, sind Rücken an Rücken aneinander verbunden und halten in deren Mitte einen wie eine elliptisch mit einem Gel oder dickflüssigem Paraffin oder medizinischen Silikon gefüllten Kissen fest. Das Kissen aus Paraffin oder Silikon sorgt dafür, dass einerseits die Auf- und Abbewegung der beiden Beutelhälften gleichmäßig und symmetrisch erfolgt und andererseits die weichen Beutel am Ende der Halbschalen nicht in die Ein- und Austrittskanäle des Blutes und der Antriebsflüssigkeit geraten. Das gelartige Kissen bewegt sich bei der rhythmischen Auf- und Ab-bewegung der beiden losen Beutelhälften wie ein stangenloser Kolben im Innenraum der Herzhälfte, der durch die Längsfäden gehalten und durch die Quer- bzw. Umfangsfäden zentriert wird.

Bei der Montage der Herzhälfte werden vorzugsweise zuerst die zylinderförmigen Anschlüsse, die Ärmel, der beiden Beutel in die entsprechenden Anschlüsse der harten Halbschalen gesteckt bis von jedem Beutel die Ärmel aus den Anschlüssen der harten Schale herausragen. Nun wird die untere Halbschale mit ihrer Dichtung versehen und die beiden harten Halbschalen werden ineinander gesteckt. Anschließend werden die beiden Beutel nacheinander mit einem gasförmigen Medium unter Druck gesetzt. Somit legen sich die beiden Beutelhälften an die Innenwände der harten Halbschale an. Die drei Anschlüsse der beiden Beutel werden über ihren runden Anschlüssen an der harten Schale zurück gestülpt. Anschließend werden die beiden Rückschlagventile durch die Ärmel in die Anschlüsse der Blutkammer gesteckt. Somit sind die Anschlüsse bereit für die Verbindung mit zwei Blutadern im oberen Bereich und einem Schlauch im unteren Antriebsbereich.

Nachdem die Vene oder Arterie oder die Aorta über ihren passenden Anschluss gestülpt wurde, sichert vorzugsweise wenigstens ein Schnellverschluss aus Kunststoff ähnlich einem Kabelbinder die Verbindung gegen ungewollte Trennung. Somit wird ein sicherer und stufenloser Übergang des Blutstromes von den Adern zur Blutkammer und umgekehrt gewährt. Die Anschlüsse der Blutbeutel, die Ärmel, können auch direkt mit der zugehörigen Vene oder Arterie angenäht werden.

Die untere Halbschale beinhaltet die Antriebskammer mit deren Anschluss und den Anschluss für das viskose Gleitmittel. Der Druck im viskosen Gleitmittel kann außerhalb des Körpers gemessen werden. Er ist ebenfalls ein Indikator für den Blutdruck innerhalb der Blutkammer.

Die Wandung der gemeinsamen Berührungsfläche der beiden Kammern, Blut- und Antriebskammer, wird vorzugsweise dreidimensional derart gestaltet, dass in der Blutkammer keine turbulente Strömung entsteht. Zu diesem Zweck werden an der äußeren Flächen der beiden Beutel Verstärkungsrippen und -streben angebracht, die sich örtlich partiell in die vorhandenen Nuten und Vertiefungen an der Innenwand der harten Schale hineinlegen und durch ihren Widerstand die Formänderung und den Bewegungsablauf der beiden zusammenwirkenden Kammern bei ihrer Arbeit bestimmen. Durch partiell unterschiedliche Wandstärken der Blut- und Antriebskammer in Umfangs- und Längsrichtung lässt sich die Elastizität und der Biegewiderstand der Wandung des Beutels derart bestimmen, dass die Abfolge deren Formänderung bei der Auf- und Ab Bewegung so optimiert wird, dass die Strömung des Blutes in die Blutkammer und aus der Blutkammer stets laminar erfolgt und die Blutkammer sich u.a. durch die zuvor beschriebene linsenförmige Verdickung vollständig und faltenfrei entleert.

Bevorzugter Weise werden im flexiblen Material, Gewebe oder Kunststoffen wie Polycarbonaturethan, PCU genannt, der beweglichen und übereinander liegenden Hälften der beiden Blut- und Antriebsbeutel auf deren Rückseite ringförmige Verdickungen wie die Zähne einer dreidimensional räumlichen Verzahnung mit Zähnen und Zahnlücken ähnlich der Breitenkreise der Erdkugel derart gebildet, dass die Zähne des einen Beutels sich beim Abrollen der beiden Rückseiten aufeinander in die Zahnlücken des anderen Beutels hineinbegeben und umgekehrt. Die so entstandenen Beutel werden Zahnbeutel genannt. Die Zähne sind in einfachster Form wie eine Triebstock-Verzahnung rund. Sie können auch im Querschnitt bzw. Normalschnitt die Form einer Evolvente oder einer Zykloide annehmen.

Der Anschluss der Antriebskammer jeder Herzhälfte wird bevorzugt mittels eines elastischen Schlauches mit einer Pumpe, vorzugsweise außerhalb des Körpers verbunden.

Ein derart arbeitendes künstliches Herz kann vorzugsweise auch außerhalb des Körpers platziert werden. In diesem Falle werden die Anschlüsse der Blutkammer durch Verlängerungsleitungen in den Körper geführt und dort mit den Venen und Arterien des alten und defekten Herzens befestigt.

Die Pumpe der Antriebsflüssigkeit besteht vorzugsweise aus zwei in Reihe oder parallel miteinander synchronisierten Zylinder-Kolben-Einheiten. Sie werden außerhalb des Körpers z.B. auf dem Brustkorb in einem geschlossenen und Wasserdichten Gehäuse an einem Korsett oder am Gürtel befestigt. Der Pumpenzylinder ist vorzugsweise waagerecht am Körper des Menschen oder des Lebewesens befestigt, während sich der Kolben entlang der Zylinderachse hin und her bewegt und die Antriebsflüssigkeit, eine Kochsalzlösung oder Kokosmilch, vom Pumpenzylinder in die Antriebskammer und umgekehrt fördert. Pro Herzhälfte existiert eine Zylinder-Kolben-Einheit. Für ein komplettes künstliches Herz werden zwei Zylinder-Kolben-Einheiten benötigt, die nebeneinander parallel oder in einer Reihe hintereinander auf einer gemeinsamen Achse in Tandembauweise oder koaxial in- und umeinander angeordnet werden. Die Anordnung der Zylinder-Kolben-Einheiten bestimmt letzendes, ob die beiden Herzhälften nacheinander oder wie ein natürliches Herz gleichzeitig das Blut in die Arterien pumpen.

Die Kolbenstangen der Pumpen werden bevorzugt durch einen Elektromotor oder in redundanter Weise durch bis zu vier Elektromotoren auf unterschiedlicher Art und Weise hin und her bewegt. Neben den bekannten Pumpenprinzipien wie Axial- und Radialkolbenpumpen, Innen- oder Außenzahnradpumpen werden die Kolbenstangen der hydraulischen Zylinder-Kolben-Einheiten vorzugsweise mithilfe einer rotierenden Scheibe durch einen oder zwei Exzenter hin und her bewegt.

Eine besonders sichere Ausführung der Hin- und Herbewegung der Kolbenstangen wird in zweckmäßigen Ausgestaltungen durch einen oder zwei endlose Spindeltriebe erzeugt. Dabei drehen ein oder zwei Elektromotoren eine Spindelwalze mit einer vorzugsweise doppelgängigen endlosen Gewindenut mit zwei Rechts- und Linksgewinde mit konstanter Winkelgeschwindigkeit stets in eine Richtung. Die Spindeltriebe bestehen aus einer vorzugsweise hohlen zylindrischen Spindelwalze, an deren Umfang sich zwei sich kreuzende Gewindenuten mit Rechts- und Linksschraubung ein- oder vorzugsweise doppelgängig derart befinden, dass deren Nutenden tangential ineinander übergehen. Damit läuft die Spindelmutter am Ende einer rechtsgängigen Nut automatisch ruck- und stoßfrei in eine linksgängige Nut und kehrt ihre Bewegungsrichtung um, während sich die Spindelwalze mit konstanter Geschwindigkeit in die gleiche Richtung weiterdreht. Die Spindelmutter besteht aus einem hohlzylinderförmigen Körper ohne Gewinde wie ein Rohrstück, das über der Spindelwalze spielarm gleitet. Innerhalb der Spindelmutter befindet sich mindestens ein Gleitstein in Form eines dreidimensional gebogenen und abgerundeten Schiffchens, das durch einen radial in der Innenwandung des hohlzylinderförmigen Körpers angebrachten Stift mit diesem verbunden ist und sich um die Achse des Stiftes in beide Richtungen um ca. ± 30° drehen kann. In jeder Spindelmutter existieren so viele Gleitsteine bzw. Schiffchen wie die Spindelwalze parallele Gewindegänge besitzt. Bei einer zweigängigen Spindelwalze besitzt beispielsweise die Spindelmutter entsprechend auch zwei Gleitsteine bzw. zwei Schiffchen, die sich in Bezug auf die Rotationsachse der Walze spiegelbildlich um 180° gegenüber liegen. Bei der Drehung der Spindelwalze übertragen die Gleitsteine bzw. die Schiffchen die rotatorische Bewegung der Spindelwalze in eine translatorische Hin- und Herbewegung der Spindelmutter, wenn die eigene Drehung der Spindelmutter verhindert wird. Die Spindelmutter ist an ihrer Außenseite mit der Kolbenstange der Pumpe oder Pumpen verbunden. Dadurch kann sich die Spindelmutter nicht um die eigene Achse drehen. Die mechanische Verbindung der Kolbenstange mit der Spindelmutter erfolgt durch eine manuell betätigte Kupplung. Diese Verbindung lässt sich in Notfällen wie z.B. beim Ausfall der elektrischen Antriebe durch den Zug an einem Knopf einfach und schnell lösen und die Kupplung auskoppeln. Danach können die Kolbenstangen und mit ihnen die Pumpen manuell betätigt werden.

Bei zwei oder mehr Antriebsmotoren und Spindeltrieben empfiehlt es sich, vorzugsweise die Spindelwalzen mechanisch, z.B. durch Zahnräder, miteinander zu koppeln.

Die natürlichen Herzklappen werden bevorzugt durch neuartige künstliche Ventile ersetzt, die in die Anschlüsse der Bluteintritts- und Blutaustrittsöffnungen in die Ärmel des Blutbeutels eingesetzt werden. Die Ventile sind insbesondere separat angefertigte und austauschbare Module, die nicht nur im künstlichen Herzen ihre Verwendung finden. Diese neuartigen Ventile können in verschiedenen Varianten aus Kunststoffen aus einem Stück gegossen, gespritzt oder in 3D-Druckverfahren gedruckt werden. Sie finden als ein ohne Fremdenergie arbeitendes Rückschlagventil Anwendung in verschiedenen Industriezweigen. Zu ihren Anwendungen gehören beispielsweise in der Medizintechnik als Ersatz für eine Herz- oder Venenklappe, im Baugewerbe als Rückschlagventil gegen Wasser- und Abwasserrückfluss der Haus- und Kanalleitungen.

Sie umfassen mehrere, mindestens drei, jedoch vorzugsweise sechs dreiecksförmige bzw. dreieckige und insbesondere gleichseitigen Lamellen, die jeweils an ihren dritten Seiten durch Gelenke, vorzugsweise Filmgelenke, an den Kanten einer mehreckigen, vorzugsweise sechseckigen Durchgangsöffnung und insbesondere einer solchen Bohrung eines Ventilringes angebracht sind. Jede Blutkammer besitzt zwei Ventile. Die Ventilringe sitzen jeweils in einem Absatz der Öffnungen der oberen Halbschale senkrecht zum Blutstrom. Der Absatz befindet sich in der Bluteintrittsöffnung an deren Ende, d.h. weiter weg von der Blutkammer und in der Blutaustrittsöffnung an deren Anfang. Damit die Ventillamellen bei Rückstrom des Blutes dicht schließen, sind beide gleichlangen Seiten der Lamellen mit je einer weiteren dreiecksförmigen Seitenlamelle drehgelenkig verbunden, dessen spitzer Winkel sich an einem Eck der mehreckigen oder sechseckigen Durchgangsöffnung bzw. Bohrung des Ventilringes befindet.

Die Ventillamellen bilden im geschlossenen Zustand eine drei- oder mehrseitige, z.B. eine sechsseitige Pyramide und im geöffneten Zustand eine Halbpyramide bis zu einem mehrseitigen, hier im Beispiel einen zwölfseitigen Zylinder. Die gegenüber der spitzen Winkel gelegenen Seiten der Seitenlamellen bilden im geschlossenen Zustand des Ventils einen Stern mit drei bis mehreren, hier im Beispiel sechs radialen Doppelflügeln. Damit die Ventillamellen bei Rückstrom des Blutes sicher und schnell schließen, befinden sich zwischen der Rückseite der Ventillamellen und dem Ventilring teilkreisförmige federnde Elemente, die stets eine Schließkraft auf die Ventillamellen ausüben.

Eine andere Variante des Ventils verzichtet auf die Seitenlamellen und befestigt die teilkreisförmigen federnden Elemente vorzugsweise jeweils an zwei benachbarten Ventillamellen.

Eine interessante vorteilhafte Variante des Ventils zeichnet sich durch eine wabenförmige offene Pyramide als Ring des Ventils aus. Die Ventillamellen legen sich im geschlossenen Zustand auf dreieckförmige Öffnungen der Seiten der Pyramide des Ventilringes. Nach Außen wird die Öffnung der Ventillamellen durch Materialüberstand auf der Rückseite der Ventillamellen begrenzt, der sich an das Rohr des Ventils anlegt.

Alle drei beschriebenen Ventilvarianten öffnen und schließen selbsttätig durch die Blutströmung ohne Fremdenergie.

Das derart konzipierte künstliche Herz oder Herzhälfte wird bevorzugt im hygienisch sauberen Reinraum vormontiert. Die Antriebskammern sowie die Pumpen werden mit Antriebsflüssigkeit gefüllt und entlüftet. Ebenfalls werden die Anschlüsse des Schmier- und Regulierungsmediums an ein Reservebehälter angeschlossen, gefüllt und entlüftet. Anschließend wird das künstliche Herz in den Körper eines Menschen oder eines anderen Lebewesens anstelle des defekten Herzens eingebaut. Zuerst werden die Hohlvenen an die Bluteintrittsöffnungen der rechten Herzhälfte angeschlossen, danach wird die Blutaustrittsöffnung der rechten Herzhälfte an die Lungenarterien angeschlossen. Anschließend werden die Lungenvenen an die Bluteintrittsöffnung und die Aorta an die Blutaustrittsöffnung der linken Herzhälfte angeschlossen. Bei Einhaltung der beschriebenen Reihenfolge ist eine nachträgliche Entlüftung der Herzkammer nicht erforderlich.

Vorzugsweise liefert ein Drucksensor im Bereich der Blutkammer den momentanen Wert des Blutdrucks. Der Druck des Schmier- und Regulierungsmediums kann an seinem Anschluss oder in seinem Behälter außerhalb des Körpers gemessen werden. Er liefert einen direkten Wert für den systolischen Blutdruck bzw. einen Wert als Indikator hierfür. Auch kleine Kameras mit Licht über Glasfaser vor den Ventilmoduln angebracht, liefern Wertvolle Informationen über den Zustand der Ventile des künstlichen Herzens.

Alle Module, die sich außerhalb des Körpers befinden, werden vorzugsweise gemeinsam mit einem Minicomputer in einem wasserdichten Gehäuse untergebracht. Zu den Sensoren, die ihre Informationen über den aktuellen Blutdruck, Herzfrequenz, Zustand der Ventile und Herzschlagvolumen an den Computer senden, gehören auch Sensoren, die den Sauerstoffgehalt des Blutes und die Atemfrequenz an den Computer weiterleiten. Der Computer berechnet aus diesen Daten die erforderliche Blutfördermenge und die erforderliche Pulsfrequenz und regelt dies.

Im Einzelnen zeigen jeweils rein schematisch:
Fig. 1 einen Längsschnitt durch eine dreidimensional ellipsoidenförmig gestaltete harten Schale einer künstlichen Herzhälfte, bestehend aus zwei Halbschalen.
Fig. 1a die Einzelheit der Steckverbindung der beiden Halbschalen einer Herzhälfte und deren Abdichtung.
Fig. 2 die Ansicht von unten auf die in Fig. 1 dargestellte linke Herzhälfte mit einem Filmgelenk (B) und zwei positiv und negativ geformten Befestigungselementen.
Fig. 3 die Ansicht auf die Unterseite von zwei gleichen Herzhälften (1R, 1L), die mit Hilfe ihrer Befestigungselemente zu einem ganzen Herzen zusammengesteckt sind.
Fig. 4 einen Längsschnitt durch die beiden gleichen Herzhälften (1R, 1L) entlang der Ebene (H) der Fig. 3.
Fig. 5a eine Herzhälfte (1) bestückt mit den Blut- und Antriebsbeuteln (12, 13), den beiden Ventilen (17A, 17V) als Ersatz für die Herzklappen, zwei optische Sensoren (21A, 21V) und einem Blutdrucksensor (22).
Fig. 5b eine Herzhälfte (1) bestückt mit den Blut- und Antriebsbeuteln (12, 13), den beiden Ventilen (17A, 17V) als Ersatz für die Herzklappen, zwei optische Sensoren (21A, 21V) und einem Blutdrucksensor (22). Die Spitzen der freien und beweglichen Hälften der Blutbeutel sind mit einem linsenförmigen Kissen (50) verbunden, das sich zwischen den beiden Endlagen annähernd parallel zu sich auf- und abbewegt und jeweils am Ende seiner Bewegung die Anschlussbohrungen der Halbschalen abdeckt.
Fig. 5c eine Herzhälfte (1) bei der die aufeinander liegenden Hälften der Blut- und Antriebsbeutel (12, 13) ring- bzw. ellipsenförmige Verdickungen besitzen.
Fig. 5d einen Teilausschnitt der aufeinander liegenden Hälften der Blut- und Antriebsbeutel (12, 13) mit den überstehenden Verdickungen (61, 62).
Fig. 6 ein ganzes künstliches Herz, bestehend aus zwei gleiche Herzhälften (1R, 1L).
Fig. 7 ein künstliches Rückschlagventil mit sechs Flügeln als Ventilklappen im geschlossenen Zustand, das bei dem künstlichen Herzen als Ersatz für eine Herzklappe zum Einsatz kommt in drei Ansichten (a, b, c).
Fig. 8 das künstliche Rückschlagventil gemäß Fig. 7 im geöffneten Zustand in drei Ansichten (a, b, c). Die sechs Ventilflügel bilden mit den zwölf Seitenflügeln ein zusammenfaltbares und schließbares Rohr.
Fig. 9 ein einfacheres künstliches Rückschlagventil im geschlossenen Zustand, das als Herzklappe dienen kann in drei Ansichten (a, b, c).
Fig. 10 das einfachere künstliche Rückschlagventil gemäß Fig. 9 im geöffneten Zustand in drei Ansichten (a, b, c).
Fig.11 ein elektrohydraulisches Pumpenaggregat mit einer Doppelkolben-Zylinder-Einheit in Tandemausführung und zwei elektromotorisch angetriebenen und beidseitig des Zylinders platzierten Spindeltrieben als redundanter Antrieb in drei Ansichten (a, b, c). Ansicht (a) ist eine Draufsicht, (b) ist eine Seitenansicht und (c) zeigt einen Längsschnitt durch die drei miteinander verbundenen Einheiten.
Fig. 12 eine Motor-Getriebe-Einheit als Antrieb für die Pumpe der Antriebskammern. Ein Elektromotor dreht eine Walze als Spindel kontinuierlich in eine Drehrichtung. Eine Spindelmutter in Form einer Hülse mit zwei innenliegenden Gleitkörpern wandert auf der Walze hin und her und treibt die Kolbenstange der Pumpen mittels einer manuell betätigten Kupplung.
Fig. 13 die Spindel des Spindeltriebes mit einer zweigängigen, rechts und links in einer geschlossenen Schleife verlaufenden Nutenspirale und einer beidseitigen Anschlusskupplung zum ein- oder beidseitigem Antrieb der Spindel.
Fig. 14 ein elektrohydraulisches Pumpenaggregat mit zwei zyklisch im Gleichtakt arbeitenden Zylinder- Kolben-Einheiten und einem elektromotorisch angetriebenen und zwischen den Pumpen platzierten Spindeltrieb als Antrieb in drei Ansichten (a, b und c). Die Ansicht (a) ist eine Draufsicht, (b) ist eine Seitenansicht und (c) zeigt einen Längsschnitt durch die drei miteinander verbundenen Einheiten.
Fig. 15 in vier Ansichten a bis d eine einfache und sichere Variante des künstlichen Rückschlagventils, das als Herzklappe, Venenklappe und in vielen anderen Industriebereichen wie in Abwasserkanälen Verwendung findet. a) zeigt das Ventil mit sechs Ventillamellen perspektivisch in der geschlossenen Stellung. b) zeigt den Schnitt durch die Mitte von zwei sich gegenüber liegenden Ventillamellen. c) zeigt das Ventil mit sechs geschlossenen Ventillamellen ohne das Gehäuse (51). d) zeigt eine Draufsicht auf das wabenförmige Gittergebilde des Ventils ohne Ventillamellen.
Fig. 16 unter a) einen Querschnitt durch die mechanische Kupplung (39) gemäß Fig. 11 senkrecht zur Kolbenstange und unter b) einen Querschnitt durch die mechanische Kupplung (63) ebenfalls senkrecht zu den Kolbenstangen gemäß Fig. 14 .
Fig. 17 zeigt eine Schnellverbindung der Venen und Arterien mit den Anschlüssen der Herzhalbschalen.

Identische Teile haben die gleiche Ziffer. Indizes mit Buchstaben kennzeichnen unterschiedliche Ausführungen desselben Elementes. Die Buchstaben haben folgenden Bezug:

| | | | |
|---|---|---|---|
| A: | Arterie | L: | bezieht sich auf die linke Seite des Patienten |
| V: | Vene | R: | bezieht sich auf die rechte Seite des Patienten |

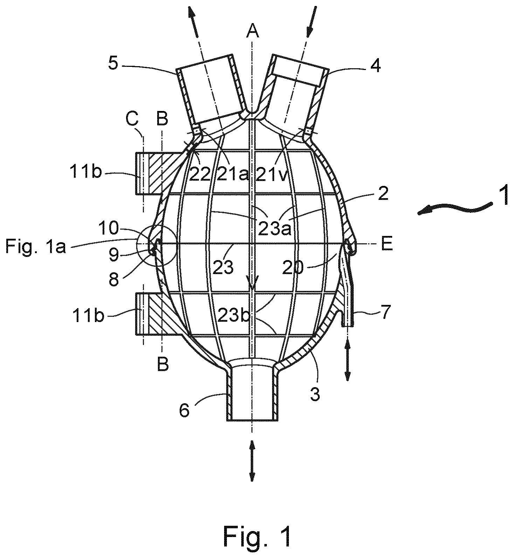

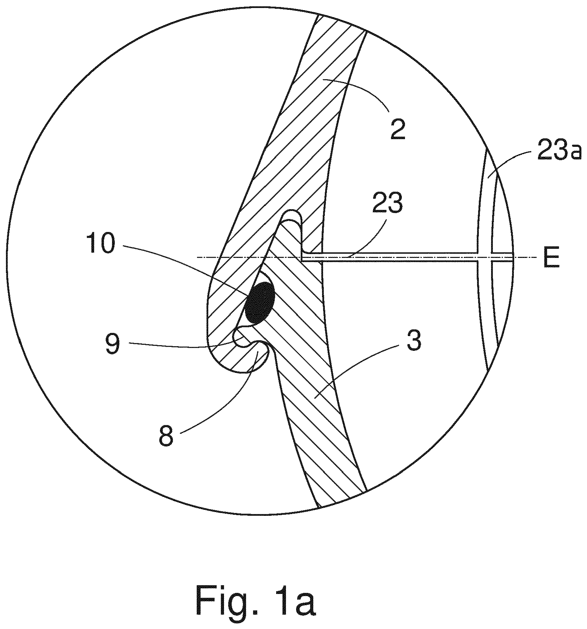

Im Folgenden erfolgt die Figurenbeschreibung:
Gemäß Fig. 1 besitzt die harte Schale einer Herzhälfte (1) die geometrische Form eines dreidimensionalen Ellipsoids, sodass der Schnitt durch die Ebene E senkrecht zur Längsachse (A) eine flache Ellipse darstellt. Die harte Schale einer Herzhälfte besteht vorzugsweise aus zwei Halbschalen, eine obere Halbschale (2) mit der Bluteintrittsöffnung (4) und der Blutaustrittsöffnung (5) und eine untere Halbschale (3) mit den Ein- und Ausgängen (6) für das Antriebsmedium und (7) für das Schmiermittel und Regulierungsmedium des Herzschlagvolumens (V). Die obere und die untere Halbschalen sind vorzugsweise mit ihren Anschlüssen aus einem biologisch verträglichen Kunststoff in Spritzguss- oder in dreidimensionalen Druckverfahren hergestellt und an der Oberfläche poliert und vorzugsweise durch Edelmetallen beschichtet. Diese besitzen, wie in Fig. 1a als Einzelheit vergrößert dargestellt, an ihren freien elliptischen Kanten nahe der Ebene (E) positive und negative Formen mit einer vorzugsweise kegelförmig schrägen Fläche, die beim Zusammenstecken aufeinander liegen. Mitten auf einer der kegelförmig schrägen Flächen der Schnittstelle der beiden Halbschalen befindet sich eine Nut und darin ein Dichtring (10), vorzugsweise ein O-Ring, der die beiden Halbschalen (2, 3) gegen Luft- und Flüssigkeit hermetisch abdichtet.

Beim Zusammenstecken der beiden Halbschalen (2, 3) geht die untere Kante (8) der oberen Halbschale (2) über die Kante (9) der unteren Halbschale (3) und umschließt die elliptische Kante rundherum fest.

Auf der Herzmittelebene (C) besitzen beide Halbschalen (2, 3) eine steckbare elastische Doppelverbindung, bestehend aus zwei übereinander liegenden positiv bzw. voll und negativ bzw. hohl geformten Zylindern (11a, 11b), die es ermöglichen, zwei gleiche Herzhälften, in Bezug auf die Herzmittelebene (C) um 180° gedreht zu positionieren und formschlüssig ineinander zu stecken. Dadurch wird ein ganzes künstliches Herz gebildet. Durch die Verjüngung der Verbindungslaschen entlang der Achse (B) entsteht ein Filmgelenk, das mehr Elastizität und Beweglichkeit zwischen den beiden Herzhälften ermöglicht. Dadurch können sich die beiden Herzhälften der individuellen Krümmung des Brustkorbs anpassen und innerhalb des Körpers relativ zueinander beweglich bleiben.

Die hier dargestellte Herzhälfte stellt die linke Hälfte eines künstlichen Herzens dar. Durch die Drehung dieser Herzhälfte um die Achse einer der Laschen (11a, 11b) um 180° entsteht eine rechte Herzhälfte. In der Bluteintrittsöffnung (4) und Blutaustrittsöffnung (5) befinden sich je ein Absatz zur Aufnahme eines Ventils, eine Bohrung (21a, 21v) zur Aufnahme einer Minikamera eines optischen Sensors oder eines Licht oder LASER-Licht reflektierenden Systems beispielsweise durch Glasfasern zwecks Überwachung und Ferndiagnose der Ventile als künstliche Herzklappen.

Der Ein- und Ausgang des Schmier- und Regulierungsmittels (7) mündet bei der Bohrung (20) in den Innenraum der Herzhälfte (1), vorzugsweise in der unteren Halbschale (3) und in den ovalen Ringspalt (23) zwischen den beiden Halbschalen (2, 3). Von dort verteilt sich das Schmiermittel- und Regulierungsmittel über die längen- und breitengradartigen Nuten (23a, 23b) auf der inneren Oberfläche der beiden Halbschalen und zwischen den beiden Beuteln, Blut- und Antriebsbeutel. Die Bohrung (22) innerhalb der oberen Halbschale (2) dient der Aufnahme eines Drucksensors, der den Blutdruck bei der Entstehung in der Blutkammer direkt und Vorort misst und per Draht an die zentrale Steuereinheit weiterleitet.

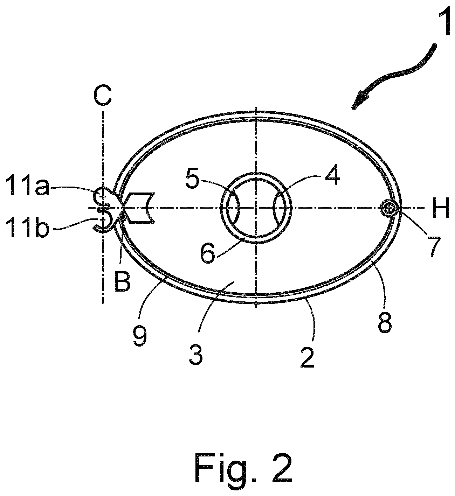

Fig. 2 zeigt die Ansicht von unten auf die linke Herzhälfte. Die äußere Ellipse ist die Kontur der oberen Halbschale (2), die mit ihrer Kante (8) die Kante (9) der unteren Halbschale (3) hintergreift. Durch den Anschluss (6) der unteren Halbschale (3) sind Teile der Bluteintrittsöffnung (4) und der Blutaustrittsöffnung (5) sichtbar.

Auf der rechten Seite der Figur 2 befindet sich an der Unterseite der Halbschale (3) auf der Ebene (H) der Anschluss (7). Die Bohrung des Anschlusses (7) mündet tangential in der elliptischen Nut (23) zwischen den beiden Halbschalen (2, 3) im Innenraum der Herzhälfte und dient der Zu- und Abfuhr eines Schmiermittels wie Paraffin zwecks Aufrechterhaltung der Dichtigkeit und Geschmeidigkeit der beiden Beutel, Reduzierung deren Reibung gegeneinander und der Regulierung des Herzschlagvolumens.

Auf der linken Seite der Figur 2 befindet sich auf der Herzmittelebene (C) an jeder Halbschale (2, 3) eine steckbare elastische Doppelverbindung (11a, 11b), bestehend aus einer Verjüngung als ein Filmgelenk (B) und zwei vorzugsweise zylindrisch positiv (11a) und negativ (11b) geformten Befestigungselementen.

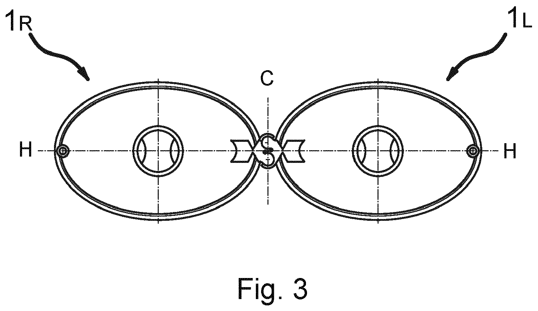

Fig. 3 zeigt die Ansicht von zwei gleichen Herzhälften (1R, 1L) von unten. Die zwei gleichen Herzhälften sind in Bezug auf die Herzmittelebene (C) um 180° gedreht angeordnet und durch Ineinanderstecken ihrer positiven (11a) in die negativen (11b) Befestigungselemente zu einem ganzen Herzen zusammengesetzt.

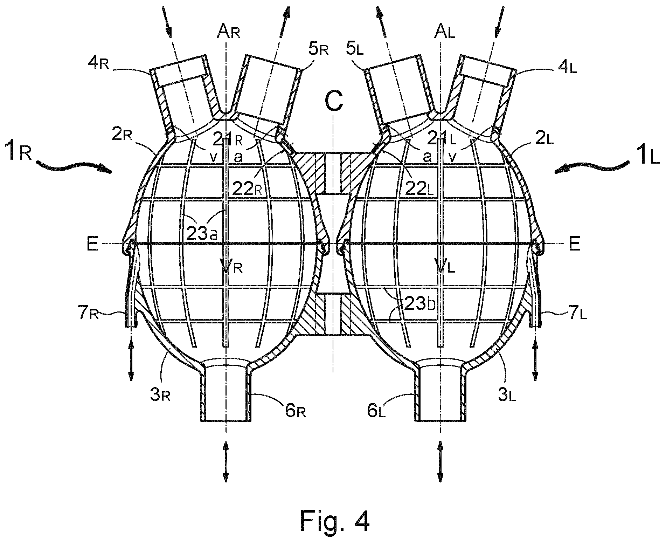

Fig. 4 zeigt einen Längsschnitt durch die harten Schalen (1R, 1L) von zwei Herzhälften eines ganzen künstlichen Herzens. Die beiden gleichen Herzhälften sind durch ihre positiven und negativen Elemente (11a, 11b) in Bezug auf die Herzmittelebene (C) um 180° gedreht zusammengesteckt.

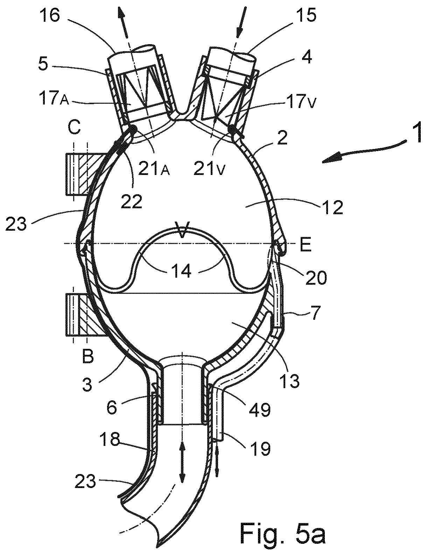

Fig. 5a zeigt eine Herzhälfte (1) bestehend aus den beiden harten Halbschalen (2, 3) im Längsschnitt. In der oberen Halbschale (2) befindet sich die Blutkammer (12) mit den beiden Ein- und Ausgängen (15, 16). Die Blutkammer (12) besteht aus einem sehr biegeelastischen Material wie Silikon oder Polyurethan, vorzugsweise wird sie jedoch aus biologischen Fasern wie der Faden der Spinne oder der Seidenraupe gewebt. Aus diesem sehr zugfesten und höchst biegeelastischen Material wird eine Blutkammer in Form eines Beutels mit dem Volumen der Herzhälfte (V) zusammen mit den beiden Ein- und Ausgängen (15, 16) als Ärmeln des Blutbeutels, bevorzugt aus einem Stück, gewebt und im geöffneten Zustand der oberen Halbschale (2) darein gesetzt. Im gesamten Bereich oder Teilbereich der oberen Halbschale, d.h. oberhalb der Äquatorebene (E), kann die Blutkammer auch an die innere Oberfläche der Halbschale geklebt werden, um sich bei der Arbeit in diesem Bereich nicht zu falten und die Kameras (21A, 21V) und den Drucksensor (22) von der Wandung nicht abzureißen. In den Bluteintrittsöffnungen Blutaustrittsöffnungen der Blutkammer (15, 16) werden Ventile (17A, 17V) als künstliche Herzklappen derart eingesetzt, dass das Material bzw. das Gewebe der Ein- und Ausgängen, die Ärmeln (15, 16) der Blutkammer zwischen den Herzklappen und den Öffnungen (4, 5) der oberen Halbschale (2) mit eingeklemmt werden. Die Ein- und Ausgängen der Blutkammer ragen soweit aus der harten Halbschale (2) hinaus, dass sie direkt mit den zugehörigen Venen und Arterien oder an Verlängerungsleitungen angenäht und/oder durch einen Klemm- und/oder Schraubverbindung und/oder Klebeverbindung verbunden werden. In den Ein- und Ausgängen der oberen Halbschalen, vorzugsweise am Ende der Blutkammer vor den Ventilen sind Kameras (21A, 21V) oder optische Sensoren und ein Drucksensor (22) eingebaut, deren Kabel (23) parallel zu den Versorgungsschläuchen (18, 19) aus dem Brustkorb nach außen zur Steuereinheit geführt werden.

In der unteren Halbschale (3) befindet sich die Druck- und Saugkammer (13). Diese Kammer wird mit ihrem Anschlussärmel (49) ebenfalls aus einem zugfesten und höchst biegeelastischen Material wie Seide oder einem Kunststofffaden wie ein Beutel aus einem Stück hergestellt. Sie hat die Form und das Volumen der Herzhälfte (1). Die Druck- und Saugkammer (13) wird vorzugsweise bis zur Hälfte an die innere Wandung der unteren Halbschale (3) angeklebt. Sie wird über den Anschluss (6) und den Schlauch (18) mit einem flüssigen Medium versorgt, das dem Blutplasma ähnlich und mit dem Blut verträglich ist. Als Antriebsmedium können auch Kochsalzlösungen oder Kokosmilch verwendet werden.

Ein Pumpenaggregat, das vorzugsweise außerhalb des Körpers, beispielsweise auf dem Brustkorb oder an einem Korsett angebracht ist, pumpt das Antriebsmedium durch einen elastischen Schlauch (18) zum Anschluss (6) in die Antriebskammer (13) hinein. Der Beutel oder Ballon der Antriebskammer drückt auf den Beutel oder Ballon der Blutkammer (12) und treibt das Blut durch das linke Ventil (17A) bei der linken Herzhälfte, wie hier dargestellt, in die Aorta oder in die Lungenschlagader bei der rechten Herzhälfte. Das Ventil (17V) der Lungenvenen bei der linken Herzhälfte oder der Hohlvenen bei der rechten Herzhälfte schließt dabei selbsttätig. Pumpt das Pumpenaggregat das Antriebsmedium aus der Antriebskammer (13) heraus, entsteht im Zwischenraum (14) zwischen den beiden Blut- und Antriebskammern ein Sog. Der Sog zieht die Blutkammer in Richtung der Antriebskammer und vergrößert ihr Volumen. Dadurch schließt das Ventil (17A) und das Ventil (17V) öffnet und lässt das sauerstoffreiche Blut der Lunge in die Blutkammer hinein fließen.

Im Zwischenraum (14) zwischen den beiden Blut- und Antriebskammern sowie in den Längs- und Quernuten (23a, 23b) auf der Innenseite der harten Schale befindet sich ein Schmiermittel wie Paraffin, das die Reibung zwischen den beiden Kammerwänden und den harten Halbschalen (2, 3) minimiert und den Stoff der beiden Kammern bzw. die Fäden der beiden Beutel dicht, geschmeidig und langlebig hält. Die Füllmenge dieses Schmiermittels bestimmt das Herzschlagvolumen der Herzhälfte und macht es bei konstantem Puls variabel. Die Regulierung der Füllmenge und der Austausch des Schmiermittels erfolgen über den Anschluss (7) und den Schlauch (19). Die Bohrung (20) des Anschlusses (7) mündet in den Zwischenraum (14) zwischen den beiden Blut- und Antriebskammern und in den ovalen Ringspalt (23) zwischen den beiden Halbschalen (2, 3) auf der Ebene (E). Von dort verteilt sich das Schmiermittel in den Nuten (23a, 23b) und zwischen den beiden Blut- und Antriebskammern. Der Anschluss (7) wird durch einen elastischen Schlauch (19) mit dem Schlauch (18) aus dem Körper durch eine Öffnung im Brustbereich vom inneren des Körpers nach außen zum Reservebehälter innerhalb des Gehäuses der Pumpen- und Steuereinheit geführt.

Von den beiden Beuteln (12, 13) haftet je eine Hälfte stets an der Innenseite seiner Halbschale (2, 3). Die andere lose Hälfte der Beutel liegen Rücken an Rücken aneinander und wandern gemeinsam und rhythmisch auf und ab.

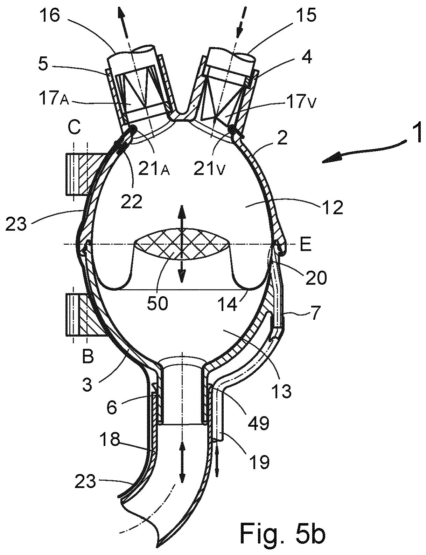

Fig. 5b zeigt eine Herzhälfte (1) bestehend aus den beiden harten Halbschalen (2, 3) im Längsschnitt, bei der zwischen den beiden Rücken an Rücken beweglich liegenden Beutelhälften ein linsenförmig und elliptisch geformtes Kissen (50) gefüllt mit einem Gel wie Paraffin oder medizinischen Silikon liegt. Das Kissen ist mittig und senkrecht zur Längsachse (A) der Herzhälfte platziert. Geführt durch die Fäden der beiden Beutelhälften bewegt sich das Gelkissen (50) wie ein stangenloser Kolben stets parallel zu sich und schließt die Öffnungen der Halbschalen in seinen Endlagen, damit kein Ballon- oder Beutelteil in die Öffnungen oder in die Ventile gerät. Das Material und die Fäden der beiden Beutel sind immer belastungs- und spannungsfrei. Auf ihnen wirken keinerlei Zug-, Druck- oder Torsionskräfte. Sie schwimmen immer zwischen den beiden Flüssigkeiten mit der Strömung hin und her bzw. auf und ab. Auf beiden Seiten der Beutel wirken stets annähernd die gleichen Kräfte. Dadurch können die verwendeten Materialien jeweils die maximal biologisch mögliche Lebensdauer erreichen.

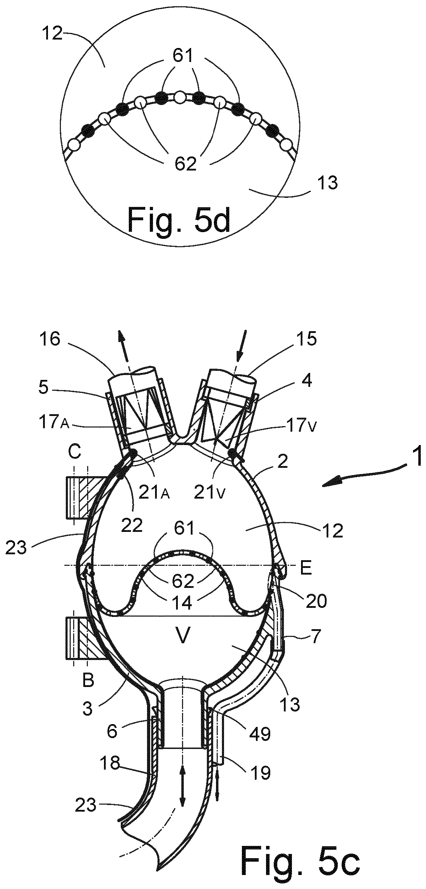

Fig. 5c zeigt eine Herzhälfte wie sie in Fig. 5a beschrieben ist. Die aufeinander liegenden Hälften der Blut- und Antriebsbeutel (12, 13) besitzen auf ihrer Rückseite kreis- bzw. ellipsenförmige Wölbungs- und Verstärkungsringe (61, 62) in Form von Zähnen, die z. B. beim Drucken der Beutel aus einem Kunststoff oder durch Einsetzen von zahnförmigen dehnungssteifen Ringen aus Kunststoffen oder aus Federstahl in das Gewebe beim Weben der Beutel erzeugt werden. Die Zähne können im Normalschnitt wie hier dargestellt kreisbogenförmig, aber auch die Form einer Zykloide oder einer Evolvente oder eine einfachere Form erhalten. Aus den Beuteln wird auf dieser Weise je ein bis zur Hälfte verzahnter Beutel. Dadurch bekommen die Beutel zahnradartige dehnungssteife kreis- oder ellipsenförmige Ringe (61, 62) an ihrer Außenseite, die sich von der Entstehung der ersten Wölbung an der Herzspitze bis zu den Ventilen (17A, 17V) Zahn in Zahnlücke liegend zusammen umbiegen und gemeinsam eine Wanderwelle erzeugen. Die Zahnringe wandern gliedweise an der Wandung der Halbschalen (2, 3) entlang bis zum Äquator und legen sich an ihren vorgegebenen Plätzen an der Wandung der Halbschalen wie die Breitenkreise der Erdkugel, ohne sich zu dehnen oder zu falten. Die steifen Zähne bzw. Ringe (61, 62) verhindern auch, dass sich die Beutel am Ende ihrer Bewegung in die Öffnungen der Herzhälften (4, 5, 6) hinein begeben können.

Fig. 5d zeigt einen kleinen Ausschnitt der beiden übereinander liegenden Blut- und Antriebsbeutel (12, 13), die hier mit einer einfachen im Normalschnitt kreisförmigen Triebstock-verzahnung versehen sind. Die Kette mit den schwarzen Gliedern (61) bzw. Zähnen stellt den Schnitt durch den Blutbeutel (12) und die Kette mit den weißen Gliedern (62) bzw. Zähnen stellt den Schnitt durch den Antriebsbeutel (13) dar.

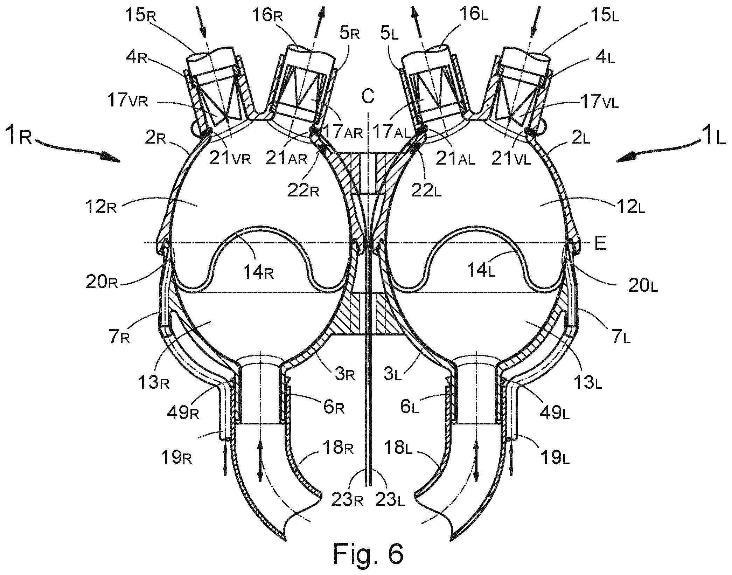

Fig. 6 zeigt ein erfindungsgemäßes künstliches Herz, bestehend aus zwei Herzhälften (1R, 1L) im Längsschnitt. Im oberen Bereich befinden sich von links nach rechts die Ventile (17VR, 17AR, 17AL, 17VL), die der Reihe nach die Trikuspidalklappe, die Pulmonalklappe, die Aortenklappe und die Mitralklappe ersetzen. Im unteren Bereich befinden sich die Anschlüsse (7R, 6R, 6L, 7L) mit den Versorgungsleitungen (19R, 18R, 18L, 19L) für das Schmier- und Antriebsmittel. Diese Versorgungsleitungen werden zusammen mit den Kabeln (23R, 23L) der Kameras (21VR, 21AR, 21AL, 21VL) und der Blutdrucksensoren (22R, 22L) aus dem Brustkorb nach außen geführt und außerhalb des Körpers an die Pumpen- und Steuereinheit angeschlossen.

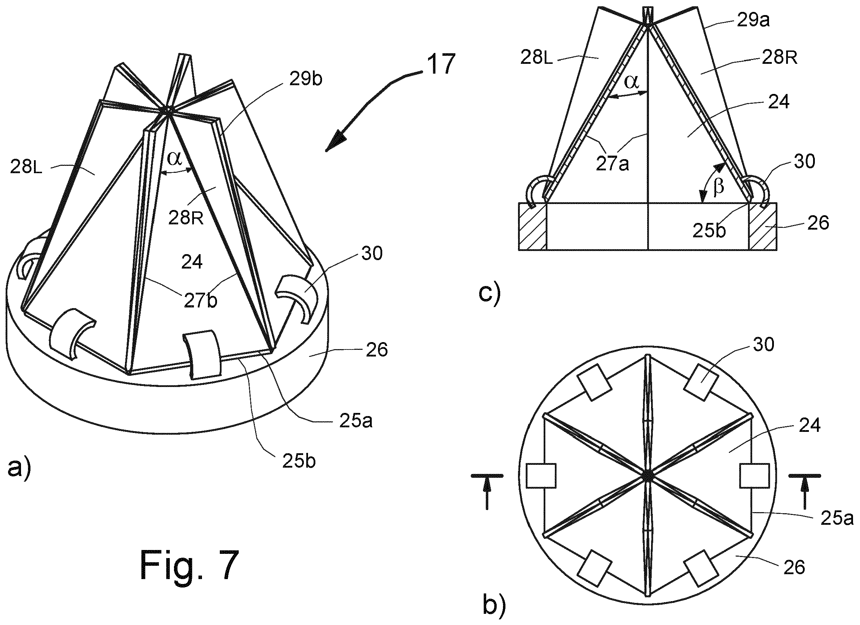

Fig. 7 zeigt ein erfindungsgemäßes Ventil (17) als eine künstliche Herzklappe im geschlossenen Zustand in drei Ansichten (a, b, c). Das Ventil besteht aus (n), mindestens drei oder mehr, vorzugsweise jedoch aus sechs dünnen Lamellen (24) aus einem festen Werkstoff wie Kunststoff, die in runder Anordnung durch Filmgelenke (25b) an einer n-eckigen Bohrung eines Ventilringes (26) schwenkbar befestigt sind. Die Ventillamellen (24), auch Hauptflügel genannt, sind dreiecksförmig mit zwei gleichlangen Seiten (27a), einem spitzen Winkel (α) dazwischen und einer kürzeren Seite (25a) gegenüber dem spitzen Winkel, die als Gelenk, wie z.B. ein Filmgelenk (25b) ausgebildet ist. Der Winkel (α) ist in der Regel kleiner als 1/n von 360°, wobei (n) die Anzahl der Lamellen ist. Jede Ventillamelle (24) neigt sich gegenüber der Ebene des Ventilringes (26) von ca. 90° im geöffneten Zustand bis zum Winkel (β) im geschlossenen Zustand. Für eine sichere Funktion und lange Lebensdauer liegt der Winkel (β) zwischen 45° und 60°. An jedem Schenkel der gleichschenkligen Ventillamellen (24) ist beidseitig je eine weitere dünne dreiecksförmige feste Seitenlamelle (28R, 28L), auch Seitenflügel genannt, durch ein Drehgelenk, in bevorzugter Weise wie hier dargestellt durch ein Filmgelenk (27b) schwenkbar befestigt. Die linke Seitenlamelle einer jeden Hauptlamelle ist ebenfalls durch ein Gelenk, vorzugsweise ein Filmgelenk (29b), mit der rechten Seitenlamelle der benachbarten Hauptlamelle schwenkbar verbunden. Jede linke Seitenlamelle ist ein Spiegelbild der rechten Seitenlamelle. Somit liegen zwischen zwei benachbarten Ventillamellen eines geschlossenen Ventils jeweils eine linke und eine rechte Seitenlamelle deckungsgleich übereinander und radial nach außen gerichtet. In dieser Position versteifen die Seitenlamellen (28R, 28L) die Ventillamellen (24) gegen Durchbiegung infolge des Blutdrucks. Darüber hinaus schließen sie den Spalt zwischen den Ventillamellen und ermöglichen ein sehr gut abdichtendes Ventil. Damit die Ventillamellen (24) stets die Tendenz zum selbsttätigen Schließen besitzen, erhalten sie optional federnde Elemente (30) beispielsweise in Form von Teilzylindern, vorzugsweise aus demselben Werkstoff. Der Blutrückstrom verbunden mit dem Blutdruck erhöht die Schließgeschwindigkeit, Schließkraft und die Dichtigkeit des Ventils. Die Abmessungen der Seitenlamellen bestimmen die Position der Hauptlamellen im geöffneten Zustand bzw. die Öffnungsweite des Ventils. Die Lage der Anbringung der Gelenke an den Kanten der harten Flügel, an der inneren oder äußeren Kante, bestimmt ob sich die Seitenflügel beim Schließen des Ventils vor oder hinter den Hauptflügeln begeben. Dies ist für die richtige Funktion des Ventils entscheidend.

Das Ventil (17) wird vorzugsweise durch das dreidimensionale Druckverfahren aus einem Stück aus Kunststoffen oder einem Gemisch aus Kunststoff- und Metall-pulver hergestellt.

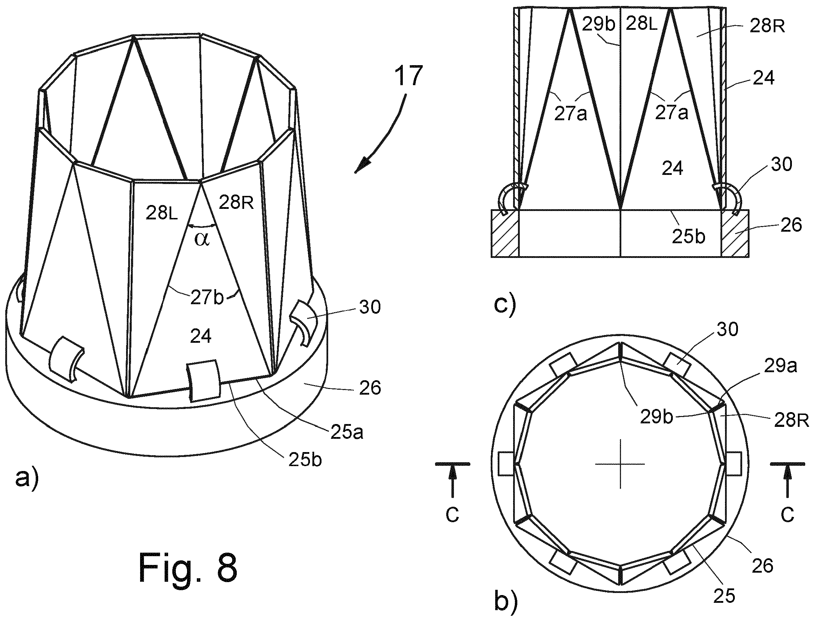

Fig. 8 zeigt das Ventil (17) im geöffneten Zustand in drei Ansichten (a, b, c). Dieses Ventil besteht aus einem Ventilring (26) mit einer sechseckigen Bohrung und sechs harten Ventillamellen (24), die an den sechs Kanten der sechseckigen Bohrung drehgelenkig angebracht sind. Die Ventillamellen sind durch Filmgelenke (25b) an den Kanten der sechseckigen Bohrung des Ventilringes (26) schwenkbar angeordnet. Federnde Elemente (30) zwischen den Ventillamellen und dem Ventilring sorgen dafür, dass sich die Ventillamellen in Ruhestellung ohne Blutstrom zum selbsttätigen Schließen neigen.

Zwischen zwei harten Ventillamellen sind jeweils zwei dreiecksförmige bzw. dreieckige harte Seitenlamellen (28R, 28L) spiegelbildlich zueinander mit der Spitze zum Ventilring (26) angeordnet. Die Seitenlamellen sind miteinander und mit den Ventillamellen durch Gelenke, vorzugsweise durch Filmgelenke (27b, 29b) schwenkbar verbunden. Die Lamellen besitzen unterschiedliche Wandstärken. Ihre Wandungen sind jeweils in der Mitte der Lamellen am stärksten ausgebildet. Die Wandstärke nimmt in Richtung der Gelenke ab. An den Ventillamellen (24) sind die Gelenke (25b) am Ventilring (26) an der inneren Kante angebracht, damit sie sich von der fast senkrechten Stellung bis zur Schließposition ca. 45° nach innen, d.h. zur Ventilmitte neigen können. Die Gelenke (27b) an den anderen zwei gleichlangen Seiten der Ventillamellen befinden sich an den nach außen gerichteten Kanten der beiden Ventil- und Seitenlamellen. Sie bewegen sich ca. 45° nach außen. Zwischen den zwei spiegelbildlich miteinander verbundenen Seitenlamellen (28R, 28L) sind die Gelenke (29b) an der Innenkante der harten Seitenlamellen angebracht, sodass die Seitenlamellen beim Schließen des Ventils selbsttätig nach innen zusammenklappen und sich komplett übereinander legen. Auch die Wandstärke der Seitenlamellen (28) ist nicht gleichmäßig. Die schmalen äußeren Kanten, die den Ausgang des Ventils bildet, sind dicker als die Spitzen, die dem Ventilring (26) näher sind. Die Gelenke an den Seitenlamellen werden vorzugsweise wie eine Hälfte eines Klavierbandes nur teilweise ausgebildet, sodass an den Ecken, wo mehrere Ventillamellen mit und ohne Seitenlamellen zusammen kommen, keine oder weniger Materialanhäufung stattfindet.

Die Ventillamellen und die Seitenlamellen besitzen an den Seiten mit Filmgelenken eine gut sichtbare und messbare Dicke mit je zwei eindeutigen Kanten. Während die eine Kante als zurückfederndes Filmgelenk ausgebildet ist, dient die andere Kante als Winkelbegrenzung des Drehgelenkes. Der räumliche Mechanismus dieses Ventils ist zwangläufig. Das bedeutet, dass das Ventil eine eindeutige und sichere Bewegungsfreiheit und Bewegungsablauf besitzt und stabil läuft.

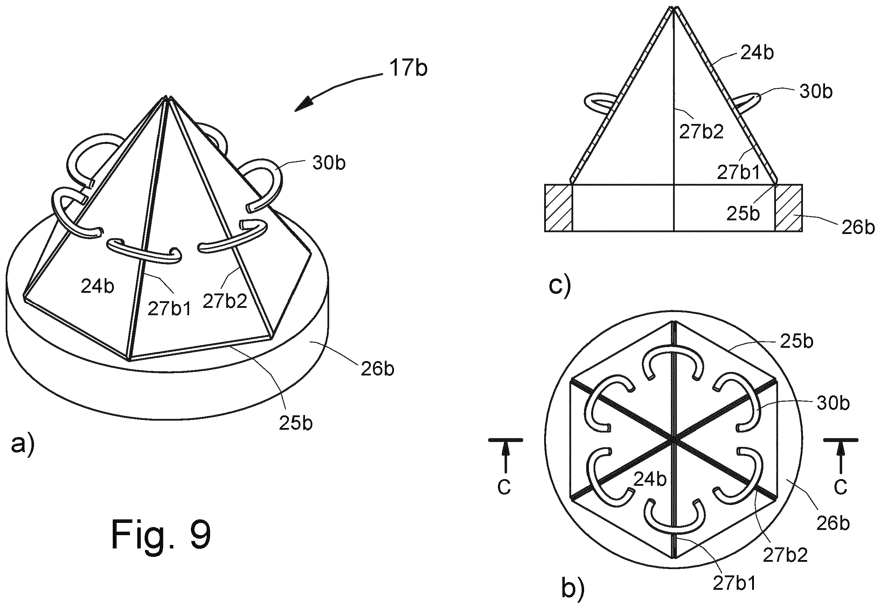

Fig. 9 zeigt eine einfachere Variante eines geschlossenen Ventils (17b) in drei Ansichten (a, b, c). Dieses Ventil besteht aus nur drei Teilen (24b, 26b, 30b). Die harten Ventillamellen (24b) sind durch Filmgelenke (25b) am Ventilring (26b) schwenkbar befestigt. Die halbkreisförmigen federnden Elemente (30b) sind bei diesem Ventil zwischen zwei benachbarten Ventillamellen angebracht. Das gesamte Ventil (17b) wird aus einem Stück aus Kunststoffen oder einem Gemisch aus Kunststoff- und Metallpulver in einem dreidimensionalen Druckverfahren hergestellt.

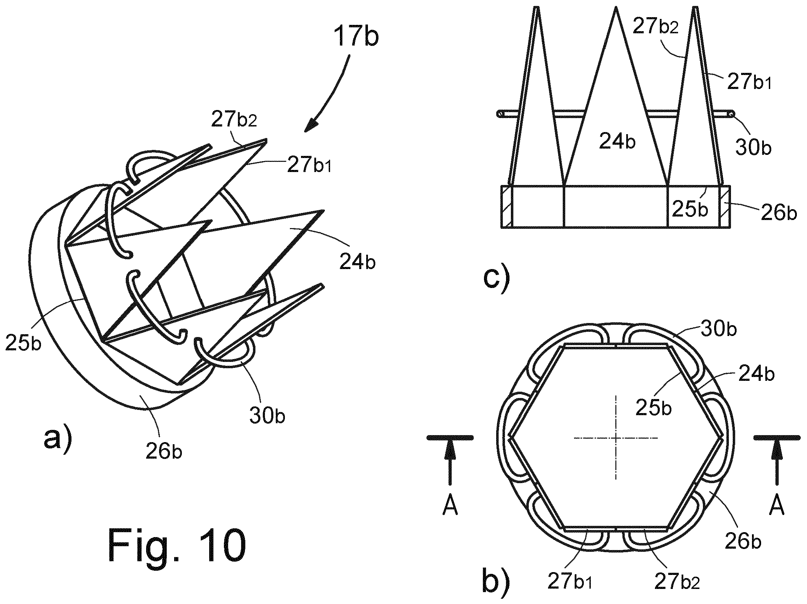

Fig. 10 zeigt das Ventil (17b) in drei Ansichten (a, b, c) im geöffneten Zustand. Die gleichlangen Seiten (27b1, 27b2) der harten Ventillamellen (24b) sind rechts und links positiv und negativ, konvex und konkav so geformt, dass sie sowohl während des Schließvorganges als auch im geschlossenen Zustand ineinander gleiten. Dadurch wird ein überschwappen und Durchschlagen einzelner Ventillamellen vermieden und die Dichtigkeit gewährleistet.

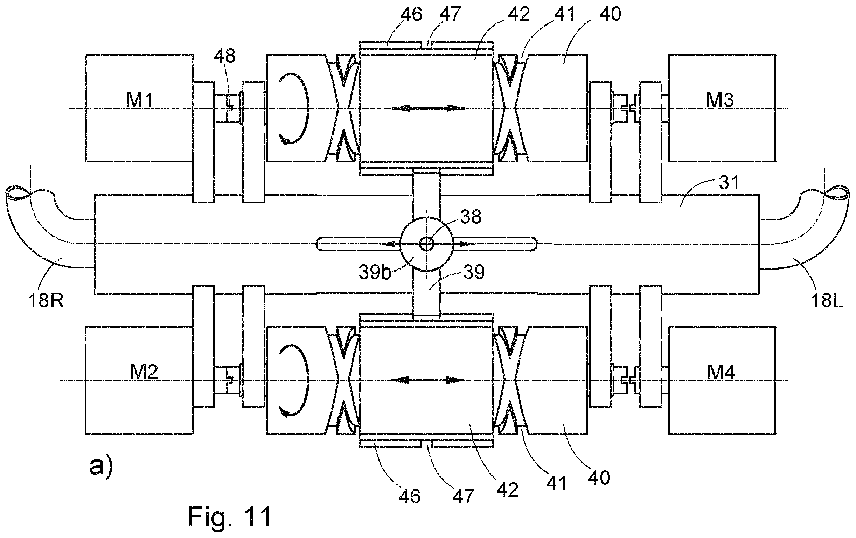

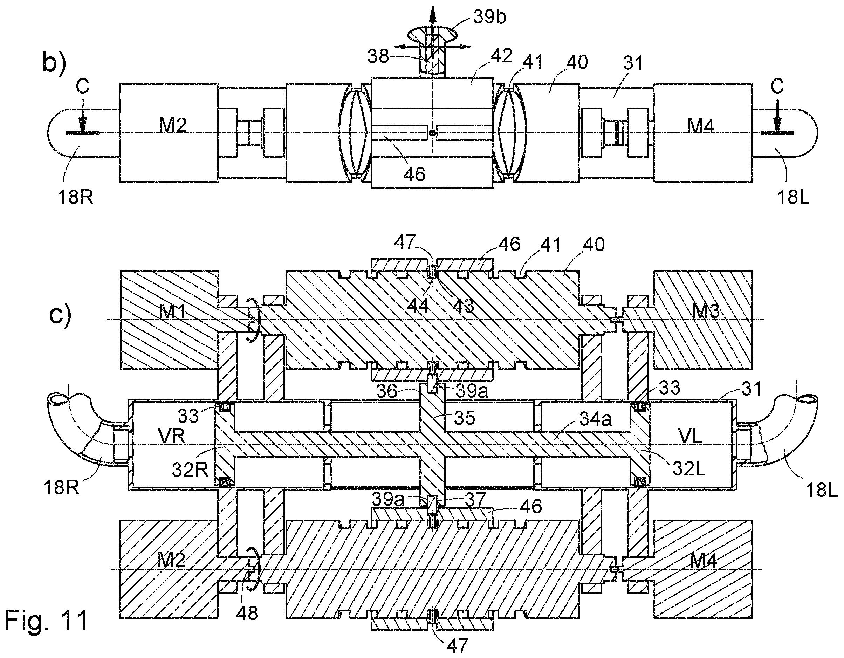

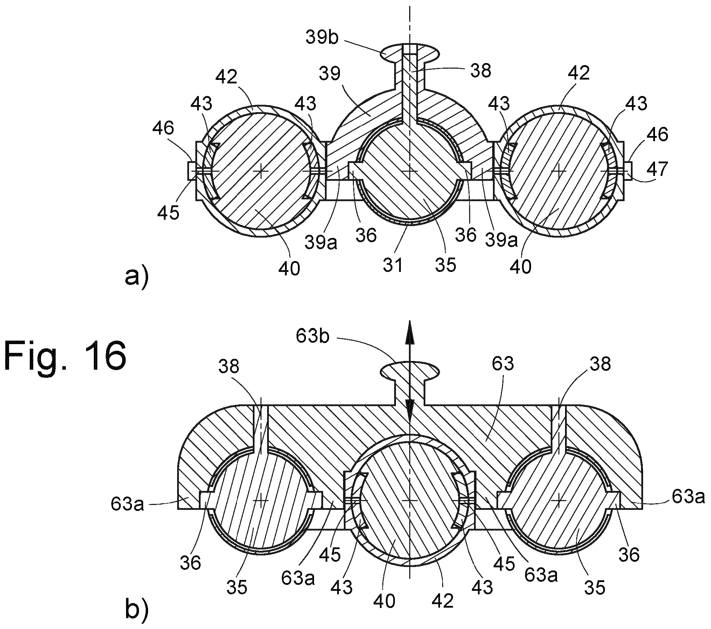

Fig. 11 zeigt ein Pumpenaggregat in drei Ansichten, Draufsicht (a), Seitenansicht (b) und Längsschnitt (c) mit einer Zylinder-Doppelkolben-Einheit (31, 32L- 32R) in Tandemausführung als Pumpe und zwei mit zwei bis zu vier Elektromotoren (M1, M2, M3, M4) angetriebenen Spindeltrieben (40, 42) als Getriebe, die beidseitig der Pumpe angeordnet sind. Die Pumpe besitzt ein zylinderförmiges Gehäuse (31), in dem die Zylinderbohrung mit dem Doppelkolben (32R, 32L), den Kolbendichtungen (33) und der gemeinsamen Kolbenstange (34a) untergebracht sind. Das Pumpengehäuse besitzt beidseitig vor jedem Arbeitsraum (VR, VL) einen Anschluss für die Antriebsflüssigkeit. Von dort gelangt die Antriebsflüssigkeit über die Leitungen (18R, 18L) abwechselnd nacheinander in die Antriebskammern der beiden Herzhälften und presst das Blut von den Blutkammern in die Lungenarterien bzw. in die Aorta. Möchte man, dass die Blutkammer der beiden Herzhälften gleichzeitig bzw. zyklisch zusammen das Blut in die Lungenarterien und Aorta pressen, ordnet man die beiden Pumpen in einfacher Ausführung, d.h. mit je einem Kolben, beidseitig der Motor-Getriebe-Einheit parallel und befestigt die Kolbenstangen durch eine manuell schaltbare Kupplung mit dem Getriebe der Antriebseinheit (siehe auch Fig. 16a). Die Kolbenstange (34a) besitzt im Zylinderraum zwei Lagerungen. Im Zwischenraum zwischen den beiden Lagerstellen befindet sich ein ringförmiges Element (35), das als Antriebsglied der Kolbenstange (34a) dient. Das Antriebsglied (35) besitzt beidseitig Kupplungselemente (36), die aus dem Gehäuse der Pumpe beidseitig durch zwei Langlöcher hinausragen und zum Antrieb durch elektromotorisch angetriebene Getriebe wie Spindeltrieb, Schneckentrieb, Kurbeltrieb, Schubkurbeltrieb, Excentertrieb, Seiltrieb, Kettentrieb, Riementrieb oder andere bekannte Antriebsmechanismen vorgesehen sind. Zusätzlich besitzt das ringförmige Antriebsglied (35) einen nach außen hoch stehenden Stift (38) als Arm, der zwischen den beiden Kupplungselementen (36) senkrecht zur Kolbenstange aus dem Pumpengehäuse durch ein Langloch hinausragt. Der Arm (38) dient der Befestigung und Führung einer manuellen Kupplung (39) und gemeinsam mit ihr der manuellen Betätigung der Kolbenstange (34a) beim Ausfall des elektrischen Antriebs.

Die Kupplung (39) besitzt einen Griff (39b) mit einem runden Knopf an dessen Ende. Mit dem Hoch- und Runterdrücken des Griffs wird die Verbindung der Kolbenstange (34a) vom Abtriebsglied (42) des Getriebes getrennt oder wieder hergestellt, indem die Spitze der Kupplung (39a) aus den Aussparungen (37, 47) der Kupplungselemente (36, 46) heraus oder hinein geschoben wird. Nach dem Entkoppeln der Kupplung (39) kann durch manuelle Betätigung des Griffs und Armes (38, 39) nach rechts und links das künstliche Herz weiter betätigt werden.

Der Antrieb der Pumpe erfolgt in einfacher Weise über einen oder zwei Elektromotoren (M1, M2) und einem Getriebe, das die rotatorische Bewegung des Elektromotors in eine hin- und hergehende translatorische Bewegung umwandelt. Das sich translatorisch bewegende Abtriebsglied (42) des Getriebes besitzt ein Kupplungselement (46) mit einer Aussparung (47) wie die Aussparung des Antriebsglieds (37) der Kolbenstange.

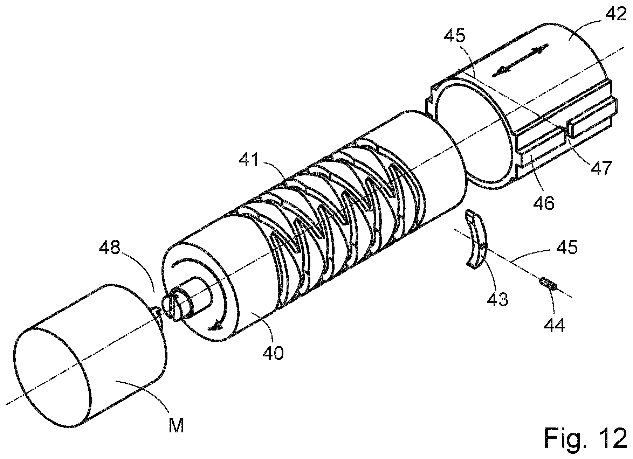

Fig. 12 zeigt eine bevorzugte Antriebsvariante der Pumpe, bestehend aus einem Elektromotor (M), der durch eine Kupplung mit Freilauf (48) einen zylindrischen Körper (40) wie eine Walze mit Nuten als Spindel mit konstanter Winkelgeschwindigkeit in eine Richtung antreibt. Auf der zylindrischen Oberfläche der Walze befinden sich zwei gegensinnig, rechts und links spiralförmig und tangential ineinander laufende Nuten (41) in doppelgängiger Ausführung. Jede rechts und links laufende Nutenspirale bildet eine in sich abgeschlossene Endlosschleife. Die beiden Endlosschleifen sind auf der Oberfläche der Spindel in Bezug auf die Rotationsachse der Walze um 180° verdreht angeordnet. Über der Walze befindet sich koaxial dazu eine Hülse (42) als ein Teil der Spindelmutter. Die Hülse besitzt beidseitig Kupplungselemente (46) in Form von überstehendem Material, das verzahnt oder mit Aussparungen (47) oder mit Bohrungen versehen sein kann. Die überstehenden Kupplungselemente (46) dienen sowohl als Verdrehsicherung der Spindelmutter als auch zum Ein- und Auskoppeln des elektrischen Antriebes von der Zylinder-Kolben-Einheit der Pumpe. Darüber hinaus besitzt die Hülse (42) eine oder zwei sich gegenüber liegende radiale Bohrungen mit der Bohrungsachse (45), eine oder zwei in den Bohrungen drehbar gelagerte Lagerbolzen (44) und einen oder zwei Gleitkörper (43) bzw. Schiffchen als Nutensteine, in denen je einer der Lagerbolzen (44) fest sitzt. Der Gleitkörper (43) besitzt eine von allen Seiten gerundete Form, die ihm gestattet, zwischen drei Seiten der Spiralnut (41) und der Hülse (42) liegend, sich relativ zu den beiden ihn umhüllenden Körpern um ca. ± 15° um die Achse (45) des Bolzens zu drehen. Er wird daher auch das Schiffchen genannt. Nach der Montage der Pumpeneinheit wird die Drehung der Hülse (42) durch die Kupplung (39) verhindert. Dadurch bewegt sich die Hülse translatorisch entlang ihrer Längsachse bis der Gleitkörper (43) am Ende seiner Nutenspirale angekommen stoß- und ruckfrei in die Gegenspirale gleitet und die Bewegungsrichtung der Hülse umkehrt. Bei der doppelgängigen Rechts- und Linksspirale und 180° Versetzung der Nutengänge laufen zwei Gleitkörper als Nutensteine in den Nutengängen spiegelbildlich und übertragen symmetrisch die Kraft von der Walze auf die Hülse.

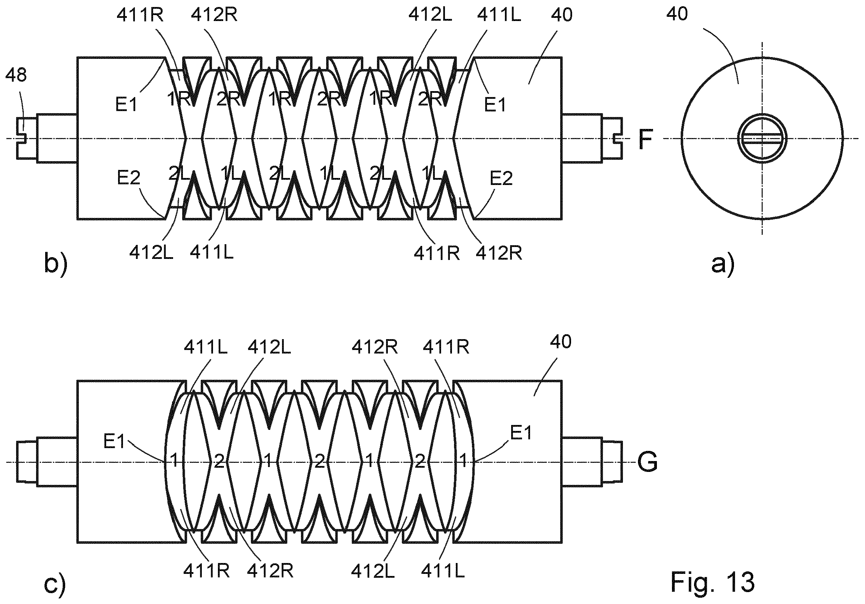

Fig. 13 zeigt die stets in eine Richtung rotierende Walze (40) mit ihrer doppelgängigen (1, 2) Spiralnuten (41). Die Nutengänge (1, 2) bilden jeweils für sich eine rechts (1R, 2R) und eine links (1L, 2L) verlaufende endlose Schleife, die am Anfang und Ende (E1, E2) ihrer Nutengänge (1R, 1L) und (2R, 2L) stoß- und ruckfrei ineinander übergehen und ihre Bewegungsrichtung wechseln. Die rechts und links Läufe einer jeden Nutenschleife (1, 2) sind in Bezug auf die Ebenen (F, G) spiegelbildlich. Die Nutenschleifen (1, 2) sind im Drehwinkel um 180° versetzt, sodass in Bezug auf die Ebene (F) die Gänge (1R) mit (2L) und (2R) mit (1L) spiegelbildlich sind.

In jeder Nutenschleife (1, 2) befindet sich ein Gleitkörper (43). Beide Gleitkörper befinden sich in jeder Position der Spindel (40) bezüglich der Rotationsachse der Spindel spiegelbildlich gegenüber. Die Spindel besitzt beidseitig Lagerzapfen und Kupplungselemente mit Freilauf (48), um gut gelagert und einseitig oder in redundanter Weise beidseitig angetrieben zu werden.

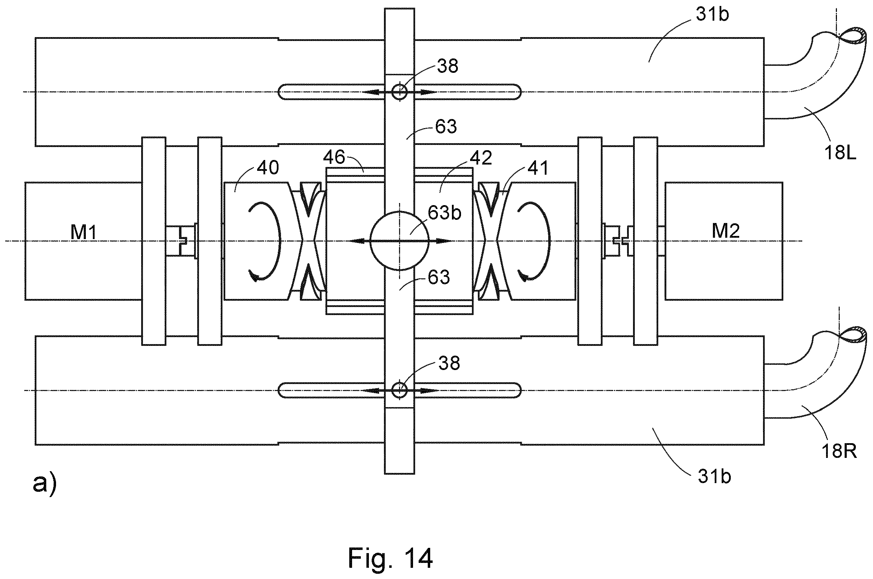

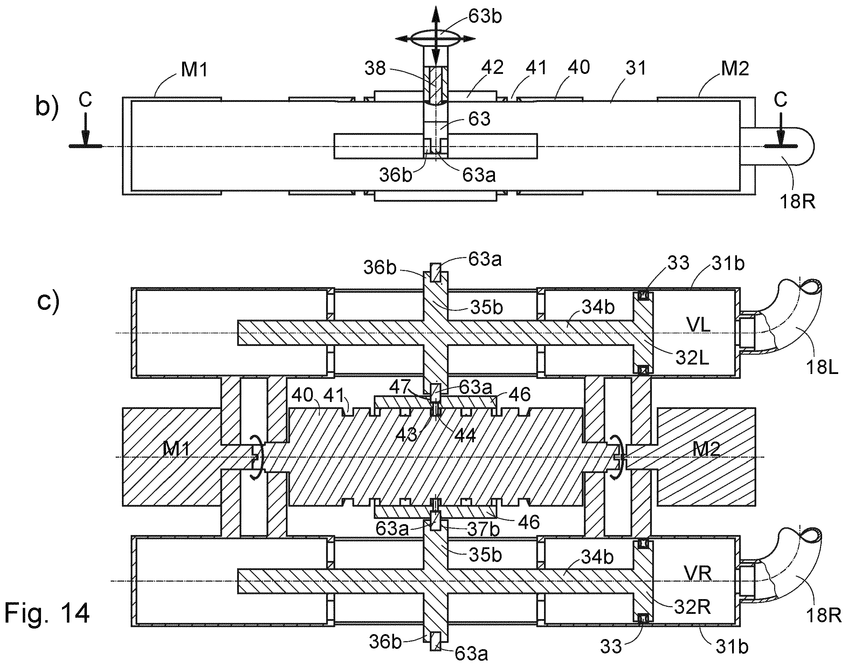

Fig. 14 zeigt ein Pumpenaggregat mit zwei einzeln und parallel angeordneten Zylinder-Kolben-Einheiten (31b, 32L, 32R) in drei Ansichten a, b, und c.

Das Pumpenaggregat besitzt ein Gehäuse (31b), mit zwei nebeneinander angeordneten Zylinderbohrungen, in denen die beiden Kolben (32R, 32L) mit ihren Kolbendichtungen (33) und Kolbenstangen (34b) untergebracht sind. Der Antrieb der Pumpen erfolgt durch einen oder zwei Elektromotoren (M1, M2) mit einem Spindeltrieb (40, 42) als Getriebe, das zwischen den beiden Zylinder-Kolben-Einheiten angeordnet ist und die rotatorische Bewegung des Elektromotors in eine hin- und hergehende translatorische Bewegung der Kolbenstangen (34b) umwandelt.

Jede Zylinderbohrung besitzt vor ihrem Kolben einen Arbeitsraum (VR, VL) mit einem Anschluss für die Antriebsflüssigkeit. Von dort gelangt die Antriebsflüssigkeit über die Leitungen (18R, 18L) im gleichen Zyklus in die Antriebskammern der beiden Herzhälften und presst das Blut von den Blutkammern in die Arterien bzw. in die Aorta. Die Kolbenstangen (34b) der einfachen Pumpe (31b) besitzen zwei Lagerungen und dazwischen je ein ringförmiges Element (35b) als Antriebsglied. Das Antriebsglied (35b) besitzt beidseitig Kupplungselemente (36b), die beidseitig aus dem Gehäuse der Pumpe durch Langlöcher hinausragen und zum Antrieb durch elektromotorisch angetriebene Getriebe wie Spindeltrieb, Schneckentrieb, Kurbeltrieb, Schubkurbeltrieb, Exzentertrieb, Seiltrieb, Kettentrieb, Riementrieb oder andere bekannte Antriebsmechanismen vorgesehen sind. Zusätzlich besitzen die ringförmigen Antriebsglieder (35b) einseitig je einen runden Stift (38) als Arm, der vorzugsweise zwischen den beiden Kupplungsspitzen (63a) senkrecht zur Kolbenstange durch ein Langloch aus dem Pumpengehäuse hinausragen. Die Arme (38) dienen der Befestigung und Führung einer manuellen Kupplung (63) und gemeinsam mit ihr der manuellen Betätigung der Kolbenstangen (34b) beim Ausfall des elektrischen Antriebs.

Die Kupplung (63) besitzt einen Griff (63b) mit einem Knopf an dessen Ende, der mittig zwischen den beiden Kolben und über der Spindelmutter (42) des Getriebes angeordnet ist. Mit dem Hoch- und Runterdrücken des Griffs (63b) wird die Bewegung der Kolbenstangen von den Kupplungselementen des Getriebes (46) getrennt oder wieder hergestellt, indem die Spitze (63a) der Kupplung (63) aus den Aussparungen (37b, 47) der Kupplungselemente (36b, 46) heraus oder in diese hinein geschoben wird (siehe auch Fig. 16b). Nach dem Entkoppeln der Kupplung (63) kann durch manuelle Betätigung des Griffs nach rechts und links das künstliche Herz durch den Patienten selbst weiter betätigt werden, was einer besseren Herzmassage entspricht als den Brustkorb zusammen zu pressen und dabei die Rippen zu brechen.

Für eine größere Sicherheit wird jede Spindel (40) des Getriebes beidseitig durch zwei Elektromotoren (M1, M2) bei Vorhandensein eines Getriebes oder (M1, M3) und (M2, M4) bei Vorhandensein von zwei Getrieben rechts und links drehend angetrieben. Die Kupplungen mit Freilauf (48) zwischen den Elektromotoren und den Spindeltrieben sorgen dafür, dass beim Ausfall eines Elektromotors die Drehung der Spindel durch den ausgefallenen Elektromotor nicht erschwert oder gar verhindert wird.

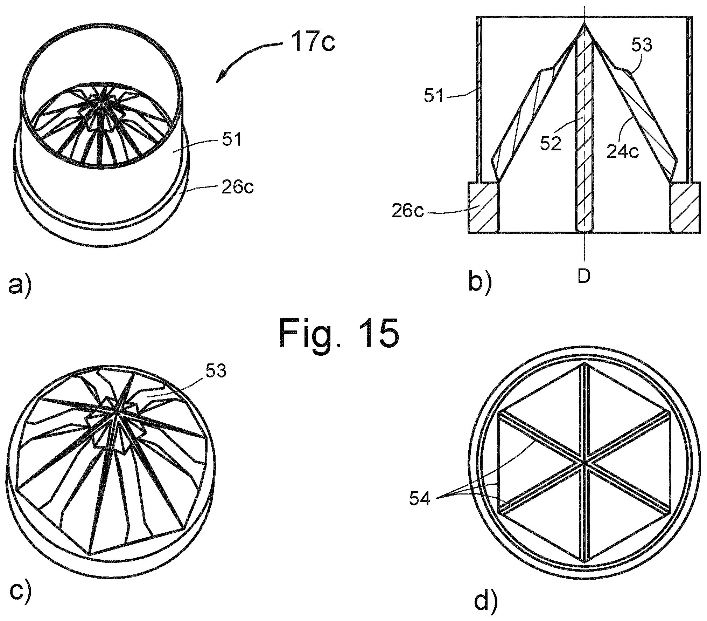

Fig. 15 zeigt ein einfach herstellbares und sicher funktionierendes Rückschlagventil (17c), das sowohl in der Medizintechnik als Herzklappe und Venenklappe verwendet werden kann, als auch in anderen Industriezweigen wie Abwasser- und Chemietechnik. In einem rohrförmig hohlen Gehäuse (51) befindet sich ein Ventilring (26c) als Absatz mit einer mindestens drei, bevorzugter Weise jedoch sechs, eckigen Durchgangsöffnung bzw. Bohrung. Jede Seite der eckigen Bohrung bildet einen hohlen geometrischen Körper mit vier dreiecksförmigen Seiten wie eine asymmetrische Pyramide, von denen eine Seite, wie in der Draufsicht d) ersichtlich, ein gleichschenkliges Dreieck bildet. Zwei Seiten sind gleich und besitzen eine gemeinsame Kante (52), die auf der Mittelachse (D) des Gehäuses liegt. Die anderen beiden Seiten sind durchgängig hohl, sodass entlang der Achse (D) ein gasförmiges oder flüssiges Medium hindurch fließen kann. Durch mehrfache Anordnung des so entstandenen hohlen Körpers um die Achse (D) gemäß der Anzahl der Seiten der eckigen Bohrung entsteht ein pyramidenförmiges Gittergebilde (54) inmitten des Rohrstücks (51), das für Gase und Flüssigkeiten beidseitig durchlässig ist. Bringt man dreiecksförmige Ventillamellen an den Seiten der eckigen Bohrung über dem Gittergebilde schwenkbar an, so entsteht ein sehr stabiles Rückschlagventil für eine Strömungsrichtung. Auf der Rückseite der Ventillamellen (24c) befinden sich buckelartige Rippen (53). Sie dienen einerseits der Verstärkung der Ventillamellen gegen Durchbiegung und andererseits dienen sie als Öffnungsbegrenzung des Ventils, indem sie sich an die Wandung des Gehäuses (51) anlegen. Fig. 16 zeigt unter a) einen Querschnitt durch die mechanische Kupplung (39a) zwischen den beiden Antriebsystemen (40, 42) und der Zylinder-Kolben-Einheit (31, 32) gemäß Fig. 11 senkrecht zur Kolbenstange (34a) und unter b) einen Querschnitt durch die mechanische Kupplung (39b) zwischen dem Antriebssystem (40, 42) und den beiden Zylinder-Kolben-Einheiten (32, 34b) gemäß Fig. 14 senkrecht zur Kolbenstange.

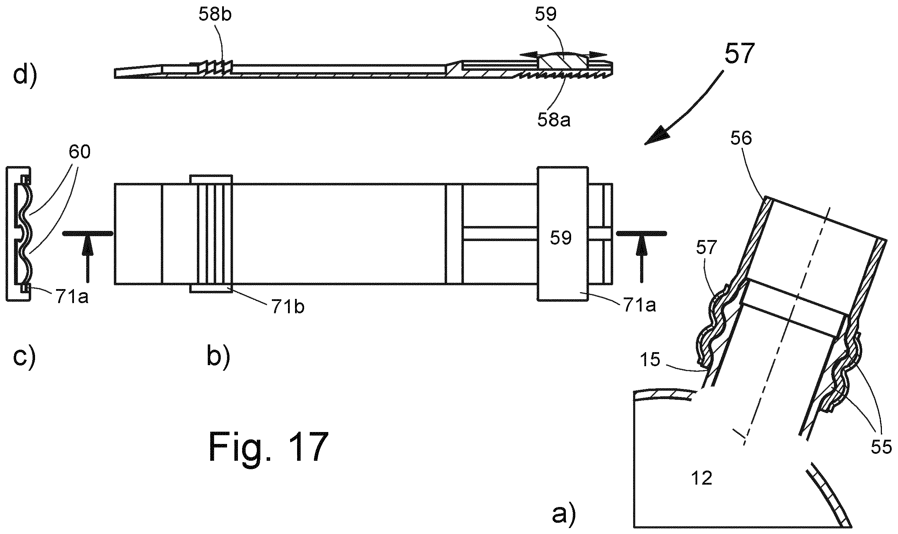

Fig. 17 zeigt eine Schnellverbindung der Venen und Arterien mit den Anschlüssen (4, 5, 6) der Herzhalbschalen. Die Anschlüsse sind gemäß Ansicht a) mit positiven Wölbungen (55) versehen. Nachdem der Ärmel (15, 16, 49) einer der Beutel (12, 13) über seinen Anschluss gestülpt wurde, und eine Vene, Arterie oder ein Schlauch darüber gesteckt wurde, wird der Schellverschluss (56) darum gelegt, durch die Widerhacken (58a, 58b) festgehalten und mit dem Schieberverschluss (59) verriegelt. Der Schnellverschluss besitzt wie in der Seitenansicht c) dargestellt, negativ geformte Vertiefungen (60) passend zu den Wölbungen (55) der Anschlüsse. Der Schieberverschluss (59) besitzt eine zweiteilige Sicherungsnase (71a, 71b) gegen unbeabsichtigtes Öffnen.

### Bezugszeichenliste:

- 1:: Herzhälfte
- 2:: obere Halbschale
- 3:: untere Halbschale
- 4:: Bluteintrittsöffnung
- 5:: Blutaustrittsöffnung
- 6:: Ein- und Ausgang des Antriebsmediums
- 7:: Ein- und Ausgang des Schmiermittels
- 8:: untere Kante der oberen Halbschale
- 9:: Kante der unteren Halbschale
- 10:: Dichtring, O-Ring
- 11a:: positive Steckverbindung der Halbschalen
- 11b:: negative Steckverbindung der Halbschalen
- 12:: Blutkammer
- 13:: Antriebskammer
- 14:: Spalt zwischen den Blut- und Antriebsbeuteln
- 15:: Ärmel = Eingang des Blutbeutels
- 16:: Ärmel = Ausgang des Blutbeutels
- 17:: künstliche Ventile (drei Varianten a, b, c)
- 18:: die Leitung bzw. der Schlauch des Antriebsmediums
- 19:: die Leitung bzw. der Schlauch des Schmiermittels
- 20:: die Bohrung bzw. der Anschluss des Schmiermittels
- 21:: Bohrung zur Aufnahme der Minikamera
- 22:: Bohrung zur Aufnahme des Drucksensors
- 23:: Ringspalt am Äquator der beiden Halbschalen
- 23a:: Längsnuten in den Halbschalen
- 23b:: Ring- bzw. Quernuten in den Halbschalen
- 24:: harte bzw. feste Ventillamelle (drei Varianten -, b, c)
- 25a:: Schmale Seite des dreieckigen Ventilflügels
- 25b:: Drehgelenk bzw. Filmgelenk zwischen Ventillamelle und Ventilring
- 26:: Ventilring (drei Varianten -, b, c)
- 27a:: Gleichschenklige Seite des Ventilflügels
- 27b:: Drehgelenk bzw. Filmgelenk zwischen Ventillamelle und Seitenlamelle
- 28:: Seitenlamelle
- 29:: Drehgelenk bzw. Filmgelenk zwischen zwei benachbarten Seitenlamellen
- 30:: Federnde Elemente, bogenförmig
- 31:: Das Gehäuse der Zylinder-Kolben-Einheit
- 32:: Der Kolben der Zylinder-Kolben-Einheit
- 33:: Kolbendichtung
- 34:: Kolbenstange (zwei Varianten a, b)
- 35:: runde Scheibe als Antriebsglied der Kolbenstange
- 36:: Kupplungsteil bzw. Kupplungselement der Kolbenstange
- 37:: Aussparung im Kupplungselement 36
- 38:: Stift als Arm oder Hebel
- 39:: Manuelle Kupplung für zwei Antriebseinheiten und eine Zylinder-Doppelkolben-Einheit in Tandembauweisemit einem Griff oder Knauf

- 39a:: Kupplungsnase
- 39b:: Kupplungsgriff bzw. -knauf
- 40:: Die Spindel, Spindelwalze
- 41:: Spiralnuten auf der Spindelwalze
- 42:: Hülse als Spindelmutter
- 43:: Schiffchen als Gleitstein
- 44:: Lagerstift, Lagerbolzen
- 45:: Achse der radialen Bohrung in der Hülsenwand
- 46:: Überstehendes Teil der Spindelmutter als Kupplungselement
- 47:: Aussparung in 46
- 48:: Kupplung mit Freilauf
- 49:: Ärmel = Anschluss des Antriebsbeutels
- 50:: konvex linsenförmiges Kissen gefüllt mit viskosem Material
- 51:: ringförmig hohles Gehäuse, Rohrstück
- 52:: gemeinsame Kante der hohlen und dreiecksförmig asymmetrischen Pyramiden
- 53:: Buckel als Verstärkungsrippen und Öffnungsbegrenzung der Ventillamellen 24c
- 54:: Das pyramidenförmige Gittergebilde
- 55:: Wölbung der Anschlüsse der Herzhälften
- 56:: Ader, Arterie oder Vene
- 57:: gewölbtes Kunststoffband als Schnellverschluss
- 58:: Widerhacken
- 59:: Schieberverschluss mit Sicherungsnase
- 60:: negativ gewölbte Oberfläche = Vertiefungen
- 61:: Ringförmig verdickte bzw. verzahnte Breitenkreise des Blutbeutels
- 62:: Ringförmig verdickte bzw. verzahnte Breitenkreise des Antriebsbeutels
- 63:: manuelle Kupplung für eine Antriebs- und zwei parallelen Zylinder-Kolben-Einheiten
- 63a:: Kupplungsspitze
- 63b:: Kupplungsgriff bzw. -knauf
- 71a:: Sicherungsnase am Kunststoffband
- 71b:: Sicherungsnase am Schieberverschluss

- A:: Längsachse der Herzhälfte
- B:: Biegeachse der elastischen Verbindung der beiden Herzhälften
- C:: Mittelebene zwischen den beiden Herzhälften
- D:: Achse des Ventilgehäuses
- E:: Die Ebene senkrecht auf die Längsachse (A) durch den Äquator der Herzhälfte
- F:: Ebene durch die Walze
- G:: Ebene durch die Walze senkrecht zu F
- H:: Ebene längs durch die Mitte des künstlichen Herzens senkrecht auf die Ebene C
- V:: Das Volumen der ganzen Herzhälfte = maximales Herzschlagvolumen
- n:: Anzahl der Seiten des Ventilringes = Anzahl der Ventillamellen (Hauptflügel der Herzklappen)

## Patentansprüche

1. Künstliches Herz zur Aufrechterhaltung und/oder Unterstützung mindestens eines Blutkreislaufes von Menschen oder anderen Lebewesen umfassend wenigstens ein und insbesondere zwei hermetisch abgedichtete Herzhälften mit je einer Blutkammer und einer Antriebskammer,
wobei jede Herzhälfte eine teilbare harte Schale (1) besitzt und
wobei die Blutkammer aus einem Beutel (12) besteht,
wobei eine erste Hälfte des Blutbeutels (12) sich wenigstens abschnittsweise bewegungslos an die Wandung eines ersten Teils der harten Schale anlegt und
wobei eine zweite Hälfte des Blutbeutels (12) dazu geeignet und ausgebildet ist, sich durch den Druck aus der Antriebskammer (13) im Wesentlichen spannungslos und vorzugsweise ohne Knickung mit einer umlaufend abrollenden Biegung wenigstens abschnittsweise und insbesondere komplett in die erste Hälfte des Blutbeutels (12) hineinzubegeben, und sich durch einen Unterdruck in der Antriebskammer (13) mit einer umlaufend abrollenden Biegung wenigstens abschnittsweise und insbesondere komplett in einen zweiten Teil der harten Schale hineinzubegeben, **dadurch gekennzeichnet,**
**dass** die Antriebskammer (13) aus einem Beutel (13) besteht, wobei eine erste Hälfte des Antriebsbeutels (13) sich wenigstens abschnittsweise bewegungslos an die Wandung der harten Schale (1) anlegt und mit seiner zweiten Hälfte sich Rücken an Rücken an den Blutbeutel anlegt, vorzugsweise spannungslos und ohne Dehnung oder Knickung den Abrollvorgang der Biegungswelle erzeugt.

2. Künstliches Herz nach Anspruch 1, **dadurch gekennzeichnet,**
**dass** auf der Rückseite der beweglichen Hälfte der Blut- und Antriebsbeutel (12, 13) breitenkreisförmige Zähne wie Wölbungen durch Materialanhäufung und/oder durch Einlegen und/oder Einarbeiten dehnungssteifer Ringe (61, 62) vorhanden sind, die sich insbesondere kettengliedförmig ineinander begeben.

3. Künstliches Herz nach einem der vorhergehenden Ansprüche, **dadurch gekennzeichnet,**
**dass** sich zwischen den beiden Beuteln (12,13) ein wenigstens ein elastisches Kissen (50) befindet, insbesondere wenigstens ein linsenförmig konvex geformtes elastisches Kissen (50) befindet.

4. Künstliches Herz nach einem der vorhergehenden Ansprüche, **dadurch gekennzeichnet,**
**dass** die Beutel (12, 13) wenigstens abschnittsweise aus einem biologischen Faden gewebt sind.

5. Künstliches Herz nach einem der vorhergehenden Ansprüche, **dadurch gekennzeichnet,**
**dass** die harte Schale (1) aus zwei Halbschalen (2, 3) besteht, die durch eine positiv und negativ formschlüssige lösbare Verbindung mit einer kegelförmigen Schnittstelle ineinander gesteckt werden.

6. Künstliches Herz nach einem der vorhergehenden Ansprüche, **dadurch gekennzeichnet,**
**dass** sich auf der Innenseite der Herzhälfte bzw. der Schale (1) Längs- und Quernuten (23, 23a, 23b) befinden, die insbesondere als dritte Kammer für die Aufnahme eines viskösen Schmier- und Abdichtmittels dienen.

7. Künstliches Herz nach einem der vorhergehenden Ansprüche, **dadurch gekennzeichnet,**
**dass** sich auf der Innenseite der Herzhälfte bzw. der Schale (1) Längs- und Quernuten (23, 23a, 23b) mit einem Anschluss nach außen befinden, die insbesondere als dritte Kammer zur Regulierung des Herzschlagvolumens dienen.

8. Künstliches Herz nach einem der vorhergehenden Ansprüche, **dadurch gekennzeichnet,**
**dass** sich an der Herzhälfte (1) positive (11a) und negative (11b) Elemente derart befinden, dass eine zweite Herzhälfte in Bezug auf ihre Längsachse (A) um 180° gedreht, an der ersten Herzhälfte befestigt wird und gemeinsam beide Blutkreisläufe des Menschen oder eines anderen Lebewesens als Blutpumpe aufrechterhalten.

9. Künstliches Herz nach einem der vorhergehenden Ansprüche, **dadurch gekennzeichnet,**
**dass** wenigstens ein Antriebsmedium wenigstens eine Flüssigkeit ist.

10. Künstliches Herz nach einem der vorhergehenden Ansprüche, **dadurch gekennzeichnet,**
**dass** das Antriebsmedium durch eine Zylinder-Kolben-Einheit in den Antriebsbeutel hinein und heraus gepumpt wird, deren Kolbenstange durch einen Spindeltrieb angetrieben wird, dessen Spindel eine doppelgängige rechts-links Endlosschleife besitzt.

11. Künstliches Herz nach Anspruch 9 **dadurch gekennzeichnet,**
**dass** das Antriebsmedium durch eine Zylinder-Doppelkolben-Einheit abwechselnd in die Antriebsbeutel hinein und heraus gepumpt wird, deren Kolbenstange insbesondere durch einen oder zwei Spindeltriebe angetrieben wird, deren Spindel eine doppelgängige rechts-links Endlosschleife besitzt.

12. Künstliches Herz nach Anspruch 9 **dadurch gekennzeichnet,**
**dass** das Antriebsmedium durch zwei Zylinder-Kolben-Einheiten gleichzeitig in die rechte und linke Antriebsbeutel hinein und heraus gepumpt wird, deren Kolbenstangen durch einen oder zwei Spindeltriebe angetrieben werden, deren Spindel eine doppelgängige, in einer endlosen Schleife rechts und links verlaufenden Spiralnut besitzt.

13. Künstliches Herz nach Anspruch 1 **dadurch gekennzeichnet,**
**dass** wenigstens ein Ein- und Auslassventil (17) der Blutkammer (12) mit dreiecksförmigen bzw. dreieckigen harten Lamellen (24) vorgesehen ist, die in runder Anordnung um eine mehreckige, vorzugsweise sechseckige Bohrung eines Ventilringes (26) durch Drehgelenke schwenkbar angeordnet sind.

14. Künstliches Herz nach Anspruch 13 **dadurch gekennzeichnet,**
**dass** an zwei Seiten der Ventillamellen (24) durch Drehgelenke (27) Seitenlamellen (28R, 28L) derart gestaltet und angeordnet sind, dass sie sich beim Schließen des Ventils hinter den Ventillamellen radial nach außen zusammenfalten, den Spalt zwischen den Ventillamellen komplett abdichten und den Ventillamellen Biegesteifigkeit verleihen.

15. Künstliches Herz nach Anspruch 14 **dadurch gekennzeichnet,**
**dass** sich anstelle der Seitenlamellen (28R, 28L) zwischen den Ventillamellen (24b) federnde Elemente wie elastische Bügel (30b) befinden.

16. Künstliches Herz nach einem der vorhergehenden Ansprüche 12 bis 14, **dadurch gekennzeichnet,**
**dass** sich am Ventilring (26c) ein offenes pyramidenförmiges Gittergebilde (54) befindet, das als Abstützung und besseren Abdichtung der Ventillamellen (24c) dient, deren Öffnungsbegrenzung durch die Anlehnung der buckelartigen Rippen (53) an das Rohrstück (51) erfolgt.

17. Künstliches Herz nach einem der vorhergehenden Ansprüche, **dadurch gekennzeichnet,**
**dass**, mindestens ein Ventil durch eine Minikamera oder mit einem optischen Sensor überwacht wird.

18. Künstliches Herz nach einem der vorhergehenden Ansprüche, **dadurch gekennzeichnet,**
**dass**, mindestens ein Drucksensor vorgesehen ist, der sich insbesondere innerhalb der harten Schale befindet, der den momentanen Blutdruck misst und an eine Steuereinheit weiterleitet.

19. Künstliches Herz nach einem der vorhergehenden Ansprüche, **dadurch gekennzeichnet,**
**dass** eine mechanische Kupplung (39, 39b) bei Bedarf die Verbindung zwischen den Antriebssystemen (M, 40, 42) und den Kolbenstangen (34, 34b) manuell entkoppelt, um die Zylinder-(Doppel)Kolben-Einheiten per Hand zu betätigen und nach Aufhebung der Störung wieder per Knopfdruck die Verbindung herzustellen bzw. die Kupplung einzukoppeln.

20. Künstliches Herz nach einem der vorhergehenden Ansprüche, **dadurch gekennzeichnet,**
**dass** die Außenseite der Anschlüsse (4, 5, 6) der Herzhälfte mit positiven Wölbungen (55) versehen sind und dass die Arterie, Vene oder der Anschlussschlauch vorzugsweise mit Hilfe wenigstens eines Schnellverschlusses (57) insbesondere mit Widerhacken (58) und/oder negativ gewölbter Oberfläche (60) sowie einer Sicherung (61) vorzugsweise locker und rutschfest befestigt werden.

## Claims

1. An artificial heart for maintaining and/or supporting at least one blood circulatory system of humans or other living beings, comprising at least one and in particular two hermetically sealed heart halves, each having a blood chamber and a drive chamber,
wherein each heart half has a divisible hard shell (1), and
wherein the blood chamber consists of a bag (12),
wherein a first half of the blood bag (12), at least in portions, lies in an immobilised state against the wall of a first part of the hard shell, and
wherein a second half of the blood bag (12) is suitable and configured for shifting into the first half of the blood bag (12), by means of the pressure from the drive chamber (13), substantially without stress and preferably without kinking by a circumferentially unrolling bending procedure, at least in portions and in particular completely, and for shifting into a second part of the hard shell, by means of a negative pressure in the drive chamber (13), by a circumferentially unrolling bending procedure, at least in portions and in particular completely,
**characterised in that**
the drive chamber (13) consists of a bag (13), wherein a first half of the drive bag (13) is placed in an immobilised state against the wall of the hard shell (1) at least in portions and with its second half back-to-back against the blood bag, and generates, preferably without stress and without strain or kinking, the unrolling procedure of the bending wave.

2. The artificial heart according to claim 1, **characterised in that**
on the rear side of the movable half of the blood and drive bags (12, 13), teeth in a shape similar to parallels of latitude are provided, like bulges by material accumulation and/or by insertion and/or incorporation of stiff rings (61, 62), particularly mutually connected like chain links.

3. The artificial heart according to any one of the preceding claims, **characterised in that**
at least one elastic pad (50) is located between the two bags (12, 13), in particular at least one lenticularly convex-shaped elastic pad (50).

4. The artificial heart according to any one of the preceding claims, **characterised in that**
the bags (12, 13) are woven at least in some portions from a biological thread.

5. The artificial heart according to any one of the preceding claims, **characterised in that**
the hard shell (1) consists of two half-shells (2, 3), which are inserted into each other by a positively and negatively interlocking detachable connection with a conical interface.

6. The artificial heart according to any one of the preceding claims, **characterised in that**
longitudinal and transverse grooves (23, 23a, 23b) are located on an inner side of the heart half or the shell (1) and serve in particular as a third chamber for receiving a viscous lubricant and sealant.

7. The artificial heart according to any one of the preceding claims, **characterised in that**
longitudinal and transverse grooves (23, 23a, 23b) with a connection outwards are located on an inner side of the heart half or the shell (1) and serve in particular as a third chamber to regulate the cardiac output.

8. The artificial heart according to any one of the preceding claims, **characterised in that**
positive (11a) and negative (11b) elements are located on the heart half (1), in such a way that a second heart half, rotated through 180° with respect to its longitudinal axis (A), is fastened to the first heart half and they together maintain, as blood pump, both blood circulatory systems of the person or another living being.

9. The artificial heart according to any one of the preceding claims, **characterised in that**
at least one drive medium is at least one liquid.

10. The artificial heart according to any one of the preceding claims, **characterised in that**
the drive medium is pumped into and out of the drive bag by a cylinder-piston unit, the piston rod of which is driven by a spindle drive, the spindle of which has a double-start right-left infinite loop.

11. The artificial heart according to claim 9, **characterised in that**
the drive medium is pumped into and out of the drive bag alternately by a cylinder-double-piston unit, the piston rod of which is driven in particular by one or two spindle drives, the spindle of which has a double-start right-left infinite loop.

12. The artificial heart according to claim 9, **characterised in that**
the drive medium is pumped simultaneously into and out of the left and right drive bag by two cylinder-piston units, the piston rods of which are driven by one or two spindle drives, the spindle of which has a double-start spiral groove running to the right and left in an infinite loop.

13. The artificial heart according to claim 1, **characterised in that**
at least one inlet and outlet valve (17) of the blood chamber (12) is provided with triangular-shaped or triangular hard lamellae (24), which are arranged in circular configuration around a polygonal, preferably hexagonal, bore of a valve ring (26) and are pivotable by pivot joints.

14. The artificial heart according to claim 13, **characterised in that**
side lamellae (28R, 28L) are designed and arranged on two sides of the valve lamellae (24) by pivot joints (27) in such a way that said side lamellae fold up radially towards the outside behind the valve lamellae when the valve closes, completely sealing the gap between the valve lamellae and increasing the bending resistance of the latter.

15. The artificial heart according to claim 14, **characterised in that**
instead of the side lamellae (28R, 28L), resilient elements such as elastic shackles (30b) are located between the valve lamellae (24b).

16. The artificial heart according to any one of claims 12 to 14, **characterised in that**
an open pyramid-shaped grid structure (54) is located on the valve ring (26c) and serves as a support and for improved sealing of the valve lamellae (24c), the opening limit of which results from the leaning of the hump-like ribs (53) against the tube piece (51).

17. The artificial heart according to any one of the preceding claims, **characterised in that**
at least one valve is monitored by a miniature camera or using an optical sensor.

18. The artificial heart according to any one of the preceding claims, **characterised in that**
at least one pressure sensor is provided, which is located in particular within the hard shell and measures the current blood pressure and forwards it on to a control unit.

19. The artificial heart according to any one of the preceding claims, **characterised in that**
a mechanical coupling (39, 39b) manually decouples the connection between the drive system (M, 40, 42) and the piston rods (34, 34b) as required in order to actuate the cylinder-(double-)piston units by hand and, once the fault has been rectified, to reestablish the connection by the push a button or reengage the coupling.

20. The artificial heart according to any one of the preceding claims, **characterised in that**
the outer side of the connections (4, 5, 6) of the heart halves are provided with positive curvatures (55), and **in that** the arteries, veins or the connection tubes are preferably secured loosely and in a non-slip manner with the aid of at least one quick-release fastener (57) in particular with barbs (58) and/or negatively curved surface (60) and a securing element (61).

## Revendications

1. Cœur artificiel destiné à maintenir et/ou à soutenir au moins une circulation sanguine d'êtres humains ou d'autres êtres vivants comprenant au moins une et, en particulier, deux moitiés de cœur hermétiquement scellées, chacune avec une chambre de sang et une chambre de propulsion ;
chaque moitié du cœur ayant une coque dure divisible (1) et
la chambre de sang étant constituée d'une poche (12),
une première moitié de la poche de sang (12) s'attachant au moins par section de manière inamovible à la paroi d'une première partie de la coque dure et
une seconde moitié de la poche de sang (12) étant adaptée et formée pour rentrer, par la pression de la chambre d'entraînement (13), essentiellement sans tension et de préférence sans pliure, avec une courbure circulaire, au moins partiellement et en particulier complètement dans la première moitié de la poche de sang (12), et rentrer dans une seconde partie de la coque dure, au moins partiellement et en particulier complètement, du fait d'une dépression dans la chambre d'entraînement (13), avec une courbure qui s'enroule périphériquement, **caractérisé en ce que**
la chambre d'entraînement (13) se compose d'une poche (13), une première moitié de la poche d'entraînement (13) se posant au moins par sections de manière inamovible sur la paroi de la coque dure (1) et générant, par sa seconde moitié dos à dos sur la poche de sang, de préférence sans tension et sans étirement ni pliage, le processus de déroulage de l'arbre de flexion.

2. Cœur artificiel selon la revendication 1, **caractérisé en ce que** :
à l'arrière de la moitié mobile des poches de sang et de propulsion (12, 13), il y a des dents circulaires larges telles que des bombements par accumulation de matériau et/ou par insertion et/ou intégration d'anneaux rigides (61, 62), qui s'entremêlent en particulier en formant une chaîne.

3. Cœur artificiel selon l'une des revendications précédentes, **caractérisé en ce**
**qu'**il y a au moins un coussin élastique (50) entre les deux poches (12, 13), en particulier au moins un coussin élastique en forme de lentille convexe (50).

4. Cœur artificiel selon l'une des revendications précédentes, caractérisé en ce
les poches (12, 13) sont tissées au moins par sections à partir d'un fil biologique.

5. Cœur artificiel selon l'une des revendications précédentes, caractérisé en ce
la coque dure (1) se compose de deux demi-coques (2, 3), qui sont insérées l'une dans l'autre par une connexion amovible positive et négative avec une interface conique.

6. Cœur artificiel selon l'une des revendications précédentes, **caractérisé en ce**
**qu'**il y a sur la face intérieure de la moitié du cœur ou de la coque (1) des rainures longitudinales et transversales (23, 23a, 23b), qui servent notamment de troisième chambre pour l'absorption d'un agent lubrifiant et isolant visqueux.

7. Cœur artificiel selon l'une des revendications précédentes, **caractérisé en ce**
**qu'**il y a sur la face intérieure de la moitié du cœur ou de la coque (1) des rainures longitudinales et transversales (23, 23a, 23b) avec une connexion vers l'extérieur, qui servent notamment de troisième chambre pour réguler le volume des battements cardiaques.

8. Cœur artificiel selon l'une des revendications précédentes, **caractérisé en ce**
**qu'**il y a des éléments positifs (11a) et négatifs (11b) sur la moitié du cœur (1), de telle sorte qu'une seconde moitié du cœur soit fixée en étant tournée de 180° par rapport à son axe longitudinal (A) à la première moitié du cœur et qu'ils maintiennent ensemble les deux circuits sanguins de l'être humain ou d'un autre être vivant sous forme d'une pompe à sang.

9. Cœur artificiel selon l'une des revendications précédentes, **caractérisé en ce**
**qu'**au moins un fluide d'entraînement est au moins un liquide.

10. Cœur artificiel selon l'une des revendications précédentes, **caractérisé en ce**
**que** le fluide d'entraînement est pompé vers l'intérieur et l'extérieur de la poche d'entraînement par une unité cylindrique-piston, dont la tige de piston est entraînée par un entraînement à broche dont la broche possède une boucle sans fin droite-gauche double.

11. Cœur artificiel selon la revendication 9, **caractérisé en ce que** :
le fluide d'entraînement est pompé alternativement dans et hors des poches d'entraînement par une unité cylindrique à double piston, dont la tige de piston est notamment entraînée par un ou deux engrenages à broche, dont la broche possède une boucle continue droite-gauche double.

12. Cœur artificiel selon la revendication 9, **caractérisé en ce que** :
le fluide d'entraînement est pompé simultanément dans les poches d'entraînement droite et gauche par deux unités à cylindre-piston dont les tiges de piston sont entraînées par un ou deux engrenages à broche dont la broche possède une rainure spiralée à double sens s'étendant à droite et à gauche, dans une boucle sans fin.

13. Cœur artificiel selon la revendication 1, **caractérisé en ce que** :
qu'au moins une vanne d'entrée et de sortie (17) de la chambre de sang (12) est prévue avec des lamelles dures en forme de triangles ou triangulaires (24) disposées de manière circulaire autour d'un alésage polygonal, de préférence hexagonal, d'un anneau de vanne (26) en pivotant grâce à des articulations rotatives.

14. Cœur artificiel selon la revendication 13, **caractérisé en ce que** :
grâce à des articulations rotatives (27), des lamelles latérales (28R, 28L) sont prévues et disposées des deux côtés des lamelles de soupape (24) de manière à ce qu'elles se replient radialement derrière les lamelles de soupape lors de la fermeture de la vanne, qu'elles colmatent complètement l'écart entre les lamelles de soupape et confèrent une rigidité à la flexion aux lamelles de soupape.

15. Cœur artificiel selon la revendication 14, **caractérisé en ce que** :
qu'au lieu des lamelles latérales (28R, 28L), il y a entre les lamelles de soupape (24b) des éléments de suspension tels que des étriers élastiques (30b).

16. Cœur artificiel selon l'une des revendications 12 à 14 précédentes, **caractérisé en ce :**
**qu'**il y a sur l'anneau de vanne (26c) une grille pyramidale ouverte (54) qui sert de support et améliore l'étanchéité des lamelles de vanne (24c) et dont la limitation d'ouverture est assurée par l'appui des nervures en forme de bosses (53) sur la pièce tubulaire (51).

17. Cœur artificiel selon l'une des revendications précédentes, **caractérisé en ce**
**qu'**au moins une soupape est surveillée par une mini-caméra ou par un capteur optique.

18. Cœur artificiel selon l'une des revendications précédentes, **caractérisé en ce**
**qu'**il est prévu au moins un capteur de pression, qui se trouve en particulier à l'intérieur de la coque dure, qui mesure la pression artérielle momentanée et la transmet à une unité de commande.

19. Cœur artificiel selon l'une des revendications précédentes, **caractérisé en ce**
**qu'**un embrayage mécanique (39, 39b) déconnecte manuellement, si nécessaire, la connexion entre les systèmes d'entraînement (M, 40, 42) et les tiges de piston (34, 34b) afin d'actionner manuellement les unités à cylindre- (double) piston et de rétablir la connexion ou l'accouplement après l'élimination du défaut par simple pression sur un bouton.

20. Cœur artificiel selon l'une des revendications précédentes, caractérisé en ce
les extrémités extérieures des raccords (4, 5, 6) de la moitié du cœur sont pourvues de bombements positifs (55) et que l'artère, la veine ou le tuyau de raccordement sont de préférence fixés de manière lâche et antidérapante de préférence à l'aide d'un système de fermeture rapide (57), en particulier avec un crochet (58) et/ou une surface à bombement négatif (60) ainsi que d'une sécurité (61).
